# EUROPEAN PATENT APPLICATION

(11) **EP 2 236 485 A1**
(43) Date of publication of application: **06.10.2010**
(21) Application number: 08864713.6
(22) Date of filing: 19.12.2008
(51) Int. Cl.: C07C 15/14, C07D 213/06, C09K 11/06, H01L 51/50

(54) **ORGANIC MATERIAL CONTAINING OLIGOPHENYLENE SKELETON AND LIGHT-EMITTING DEVICE USING THE SAME**

(30) Priority: 25.12.2007 JP 2007332855
(71) Applicant: National University Corporation Nagoya University, Aichi 464-8601 (JP); Kyushu University, National University Corporation, Higashi-ku Fukuoka-shi Fukuoka 812-8581 (JP); Central Glass Company, Limited, Yamaguchi 755-0001 (JP)
(72) Inventor: YAMAGUCHI, Shigehiro, Aichi 464-8601 (JP); WAKAMIYA, Atsushi, Aichi 464-8601 (JP); ADACHI, Chihaya, Fukuoka 812-8581 (JP); YAHIRO, Masayuki, Fukuoka 812-8581 (JP); ENDO, Ayataka, Fukuoka 812-8581 (JP); IDE, Toshihisa, Yamaguchi 755-0001 (JP); SHINMEN, Masutaka, Yamaguchi 755-0001 (JP)
(74) Representative: Manitz, Finsterwald & Partner GbR
(86) International application number: PCT/JP2008/073222
(87) International publication number: WO 2009/081873

(57) **Abstract**

[Object]

To provide a wide band-gap material capable of forming a stable amorphous thin film and an organic electroluminescent device using such a compound and having a high light emission efficiency.

[Solution]

It has been found that a novel oligophenylene derivative, which is applicable as an organic electroluminescent material, can be produced efficiently using a cross-coupling reaction. It has also been found that a highly-efficient blue phosphorescent light-emitting device can be produced using this compound. The present invention is based on these findings.

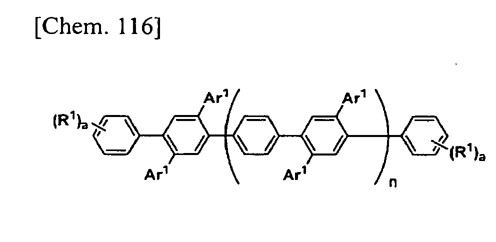

## Description

### Technical Field

The present invention relates to an organic π-electron system material having an oligophenylene skeleton and a light-emitting device using the organic material.

### Background Art

Attention is being given to organic light-emitting devices, notably organic electroluminescent devices having electroluminescent functions, for next-generation flat panel displays. The use of these organic electroluminescent devices enables full-color high-resolution displays that feature low power consumption, wide viewing angle, self emission and quick response.

The conventional type of the organic electroluminescent device mainly utilizes fluorescent light emissions. The organic electroluminescent device has a light-emitting layer arranged between electrodes so that electrons and positive holes are injected from the respective electrodes into the light-emitting layer and recombined together at a certain rate in the light-emitting layer to form excitons and produce light emission by decay of the excitons from the excited states to the ground state. Herein, the excited states are classified as a singlet excited state in which electron spins are opposite and a triplet excited state in which electron spins are parallel. The fluorescence is light emission derived only from the singlet excited state. Based on the simple quantum-mechanical reasoning that the generation rate of the singlet excited state and the triplet excited state is 1:3, the internal quantum efficiency of the organic electroluminescent device utilizing fluorescence is assumed to be maximum 25%. It means that the fluorescent organic electroluminescent device does not use 75% of the excitation states for light emission.

Further, the organic electroluminescent device generally contains an organic material with a refractive index (n) of about 1.6 to 1.7 and thus shows an external light extraction efficiency of ηₑₓₜ = 1/(2n²) ≈ 0.2, i.e., on the order of 20% according to the laws of reflection and refraction of classical optics. The external quantum efficiency of the organic electroluminescent device utilizing fluorescence is assumed to be about maximum 5% as determined by multiplying the internal quantum efficiency (25%) by the light extraction efficiency (20%).

It is thus necessary to utilize phosphorescence i.e. light emission from the triplet excited state, which occupies 75% of the excited states, in order to further improve the external quantum efficiency of the organic electroluminescent device. The utilization of phosphorescence could lead to about maximum 20% improvement in external quantum efficiency.

For the above reasons, the development of phosphorescent organic electroluminescent devices has recently been promoted. There are reports that, with the use of a phosphorescent material, the external quantum efficiency of the conventional fluorescent device can be improved to a level exceeding the theoretical limit of 5% and reach a high efficiency level of 19% in the case of green light emission (See Non-Patent Documents 1 and 2).

In terms of high-efficiency light emissions, phosphorescent light-emitting materials utilizing phosphorescence are being intensively researched and developed. Green and red phosphorescent light-emitting materials of high color purity are already reported. Organic electroluminescent devices with blue phosphorescent light-emitting materials are also reported in e.g. Non-Patent Documents 3 to 5.
Non-Patent Document 1: J. Appl. Phys., Vol. 90 (2001), P. 5048
Non-Patent Document 2: Appl. Phys. Lett., Vol. 79 (2001), P. 156
Non-Patent Document 3: Appl. Phys. Lett., Vol. 79 (2001), P. 2082
Non-Patent Document 4: Appl. Phys. Lett., Vol. 82 (2003), P. 2422
Non-Patent Document 5: Appl. Phys. Lett., Vol. 83 (2003), P. 569
Patent Document 1: Japanese Laid-Open Patent Publication No. 7-157473
Patent Document 2: Japanese Laid-Open Patent Publication No. 2002-212181
Patent Document 3: Japanese Laid-Open Patent Publication No. 2005-129310
Patent Document 4: Japanese Laid-Open Patent Publication No. 11-283746

### Disclosure of the Invention

As mentioned above, various types of organic electroluminescent devices have heretofore been developed. There are however only limited examples of phosphorescent light-emitting devices succeeding in blue light emissions that require wide band-gap material excitation.

For example, Patent Documents 1 to 4 are directed to fluorescent light-emitting devices and are not intended to utilize phosphorescence. Although Non-Patent Documents 1 and 2 are directed to light-emitting devices that utilize phosphorescence to attain a high energy efficiency, these devices are provided with medium band-gap materials for green light emissions and has not succeeded in blue light emissions.

On the other hand, Non-Patent Documents 3 to 5 teach that blue phosphorescence can be observed by the use of a carbazole derivative (represented by the following formula), called "4,4'-biscarbazolylbiphenyl (hereinafter abbreviated as CBP)", as a host material for a phosphorescent dopant. 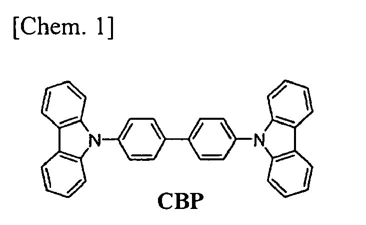 However, it cannot be said that the organic electroluminescent device using CBP as the host material has a high energy efficiency. As is seen in Non-Patent Document 3, the quantum efficiency of such an EL device is merely on the order of 5.7% and cannot be said to be high. Further, it is difficult in some cases to form a stable thin film of CBP because of its high crystallinity.

Under these circumstances, there has been a demand to develop an organic electroluminescent device and, more specifically, a highly-efficient and highly-durable host material for blue phosphorescent emission and an organic electroluminescent device using such a material.

As a result of extensive researches made to overcome the above problems, the present invention have found a novel organic electroluminescent device having a pair of electrodes and at least one organic light-emitting layer arranged between the electrodes, wherein the organic light-emitting layer contains an oligophenylene derivative represented by the general formula (1) 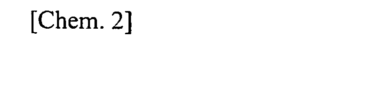 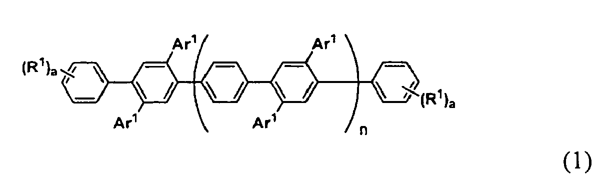 where Ar¹ each independently represents an oligophenyl group of the following formula (1a); *n* is 0 or 1; R¹ each independently represents an C₁-C₆ alkyl group, a C₁-C₆ fluoroalkyl group or a halogen atom; and *a* is each independently an integer of 0 to 5

[Chem. 3] -(Ar')_{b}-(Ar")_{c} (1b)

where Ar' each independently represents a divalent to hexavalent aromatic ring which may have a substituent; Ar" each independently represents a phenyl group which may have a substituent; each substituent of Ar' and Ar" can be located at any position on the aromatic ring and is selected from the group consisting of a C₁-C₆ alkyl group, a C₁-C₆ fluoroalkyl group and a halogen atom; each of Ar' and Ar" may have one to four nitrogen atoms as a heteroatom in the aromatic ring; *b* is an integer of 1 to 4; *c* is an integer of 1 to 5; Ar' and Ar' or Ar' and Ar" are bonded to each other by a single C-C bond between carbon atoms of the respective aromatic rings; *c* number of Ar" can be bonded to Ar' in straight chain form or in branched chain form or can be directly bonded to Ar'.
It has been found that this oligophenylene derivative is a novel compound having a basic skeleton consisting of carbon and hydrogen atoms and optionally nitrogen and/or halogen atoms and can be efficiently produced using known compounds as raw materials by combination of reaction processes including a coupling reaction process

It has also been shown that this oligophenylene derivative serves as a higher performance host material in an organic electroluminescent device than a conventional material and, in particular, suitably serves as a host material of a blue phosphorescent organic electroluminescent device so that the blue phosphorescent organic electroluminescent device using the oligophenylene derivative can attain a higher energy efficiency than that of the conventional type using CBP as a host material.

In the present invention, the basic skeleton of the oligophenylene derivative is formed of benzene or pyridine rings with other benzene or pyridine rings substituted on the ortho positions of the benzene ring skeleton. All of the aryl groups are slightly twisted out of the molecular plane, thereby weakening the spread of the π-electron resonance structure (π conjugation) of the oligophenylene derivative. As a result, the band gap between the highest occupied molecular orbit (HOMO) and the lowest unoccupied molecular orbit (LUMO) of the oligophenylene derivative increases sufficiently. It is thus considered that the oligophenylene derivative can efficiently serve as the blue phosphorescent host material.

In general, it often becomes more difficult to form an excited state of the material as the band gap of the material increases. However, the oligophenylene derivative of the present invention, when used as a host material, shows a significantly higher energy efficiency that that of a conventional CBP. It is considered that these favorable properties of the oligophenylene derivative result from the combined effect of:
(1) increasing the flexibility (amorphous nature) of the molecule due to a twisted molecular structure and thereby improving the durability of the entire light-emitting layer; and (2) weakening the π conjugation of the molecule due to non-planarity of the molecular structure and thereby increasing the band gap of the molecule effectively.

The present inventors have further found particularly preferable structures of the oligophenylene derivative of the formula (1) and preferable embodiments and conditions of use of the oligophenylene derivative of the formula (1). The present invention is based on the above findings.

In other words, the present invention provides an organic material having an oligophenylene skeleton and a light-emitting device using the organic material according to the following features 1 to 22.

### [Feature 1]

An oligophenylene derivative represented by the general formula (1) 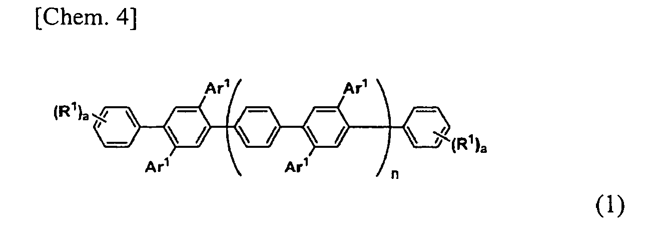 where Ar¹ each independently represents an oligophenyl group of the following formula (1a); *n* is 0 or 1; R¹ each independently represents an C₁-C₆ alkyl group, a C₁-C₆ fluoroalkyl group or a halogen atom; and *a* is each independently an integer of 0 to 5

[Chem. 5] - (Ar') _{b} - (Ar") _{c} (1a)

where Ar' each independently represents a divalent to hexavalent aromatic ring which may have a substituent or substituents; Ar" each independently represents a phenyl group which may have a substituent or substituents; each substituent of Ar' and Ar" can be located at any position on the aromatic ring and is selected from the group consisting of a C₁-C₆ alkyl group, a C₁-C₆ fluoroalkyl group and a halogen atom; each of Ar' and Ar" may have one to four nitrogen atoms as a heteroatom in the aromatic ring; *b* is an integer of 1 to 4; *c* is an integer of 1 to 5; Ar' and Ar' or Ar' and Ar" are bonded to each other by a single C-C bond between carbon atoms of the respective aromatic rings; *c* number of Ar" can be bonded to Ar' in straight chain form or in branched chain form or can be directly bonded to Ar'.

### [Feature 2]

The oligophenylene derivative according to Feature 1, wherein Ar¹ is an oligophenyl group of the following formula (1b)

[Chem. 6] - (Ar') _{b}- (Ar") (1b)

where Ar', Ar" and *b* are the same as defined in the formula (1a).

### [Feature 3]

The oligophenylene derivative according to Feature 1, wherein Ar¹ is an oligophenyl group of the following formula (1c) 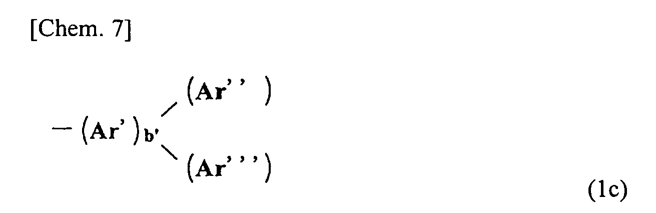 where Ar' and Ar" are the same as defined in the formula (1a); A"' is the same as Ar"; and *b'* is the same as *b.*

### [Feature 4]

The oligophenylene derivative according to any one of Features 1 to 3, wherein the oligophenylene derivative is a terphenyl derivative of the general formula (2) 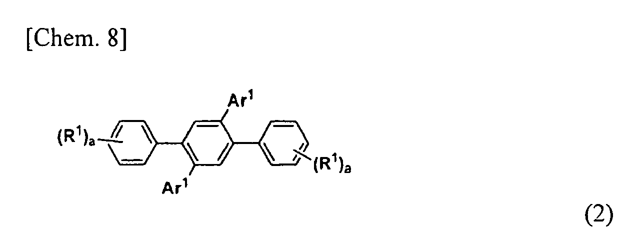 where Ar¹ and R¹ are the same as defined in the general formula (1).

### [Feature 5]

The terphenyl derivative according to Feature 4, wherein the terphenyl derivative is represented by the general formula (3) 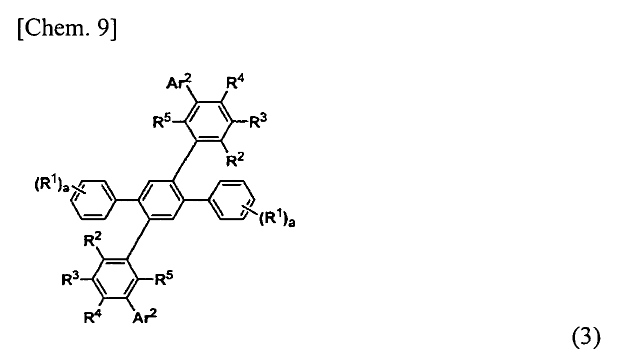 where R¹ and *a* are the same as defined in the formula (1); R², R³, R⁴ and R⁵ each independently represent a C₁-C₆ alkyl group, a C₁-C₆ fluoroalkyl group or a halogen atom; Ar² represents a phenyl group which may have a substituent or substituents; each substituent of Ar² can be located at any position on the aromatic ring and is selected from the group consisting of a C₁-C₆ alkyl group, a C₁-C₆ fluoroalkyl group and a halogen atom; and Ar² may have one to four nitrogen atoms as a heteroatom in the aromatic ring.

### [Feature 6]

The terphenyl derivative according to Feature 4, wherein the terphenyl derivative is represented by the general formula (4) 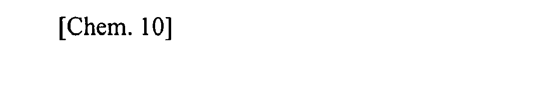 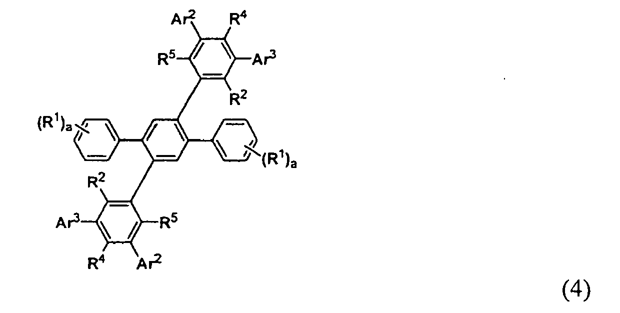 where R¹ and *a* are the same as defined in the formula (1); R², R⁴ and R⁵ each independently represent a C₁-C₆ alkyl group, a C₁-C₆ fluoroalkyl group or a halogen atom; Ar² and Ar³ each independently represent a phenyl group which may have a substituent or substituents; each substituent of Ar² and Ar³ can be located at any position on the aromatic ring and is selected from the group consisting of a C₁-C6 alkyl group, a C₁-C₆ fluoroalkyl group and a halogen atom; and each of Ar² and Ar³ may have one to four nitrogen atoms as a heteroatom in the aromatic ring.

### [Feature 7]

The terphenyl derivative according to Feature 4, wherein the terphenyl derivative is represented by the general formula (5) 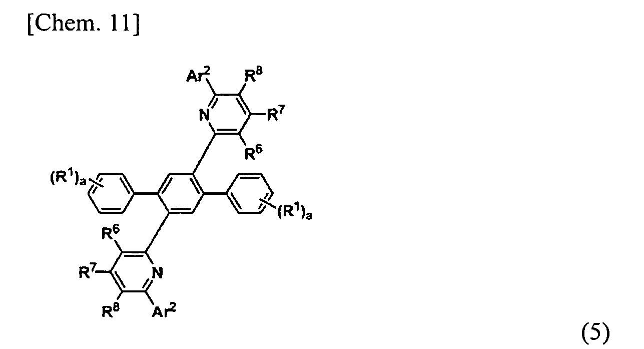 where R¹ and *a* are the same as defined in the formula (1); Ar² is the same as defined in the formula (3); and R⁶, R⁷ and R⁸ each independently represent a C₁-C6 alkyl group, a C₁-C₆ fluoroalkyl group or a halogen atom.

### [Feature 8]

The terphenyl derivative according to Feature 4, wherein the terphenyl derivative is represented by the general formula (6) 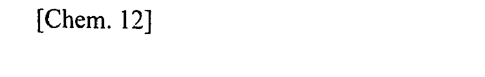 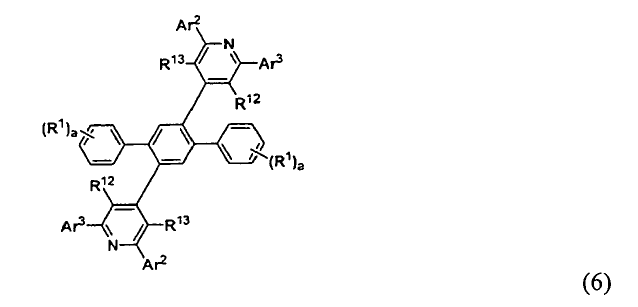 where R¹ and *a* are the same as defined in the formula (1); Ar² and Ar³ are the same as defined in the formula (5); and R¹² and R¹³ each independently represent a C₁-C₆ alkyl group, a C₁-C₆ fluoroalkyl group or a halogen atom.

### [Feature 9]

An organic electroluminescent device, comprising a pair of electrodes and at least one organic light-emitting layer arranged between the electrodes, wherein the organic light-emitting layer contains an oligophenylene derivative represented by the general formula (1) 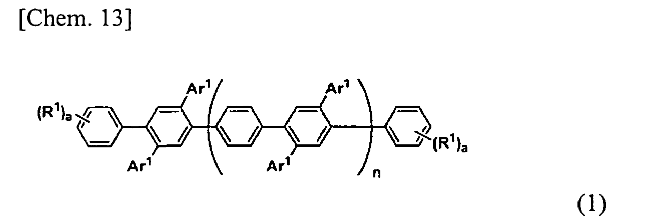 where Ar¹, R¹, *a* and *n* are the same as defined in the above formula (1).

### [Feature 10]

The organic electroluminescent device according to Feature 9, wherein Ar¹ is an oligophenyl group of the following formula (1b)

[Chem. 14] - (Ar') _{b}- (Ar") (1b)

where Ar', Ar" and *b* are the same as defined in the above formula (1a).

### [Feature 11]

The organic electroluminescent device according to Feature 9, wherein Ar¹ is an oligophenyl group of the following formula (1c) 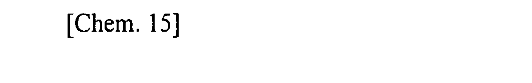 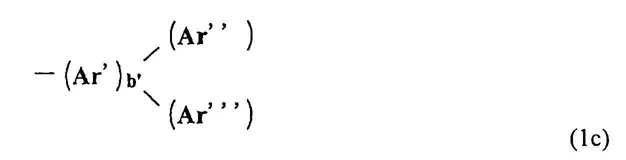 where Ar' and Ar" are the same as defined in the formula (1a); A"' is the same as Ar"; and *b*' is the same as *b*.

### [Feature 12]

The organic electroluminescent device according to any one of Features 9 to 11, wherein the organic light-emitting layer contains a host material and a blue phosphorescent dopant material; and the host material is the oligophenylene derivative.

### [Feature 13]

The organic electroluminescent device according to Feature 12, further comprising a positive hole transfer layer, an exciton block layer and an electron transfer layer.

### [Feature 14]

The organic electroluminescent device according to any one of Features 9 to 13, wherein the oligophenylene derivative is a terphenyl derivative of the general formula (2) 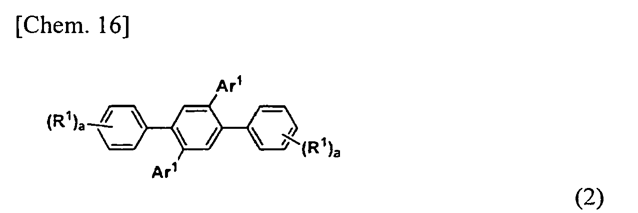 where Ar¹, R¹ and *a* are the same as defined in the above formula (2).

### [Feature 15]

The organic electroluminescent device according to Feature 14, wherein the terphenyl derivative is represented by the following formula (3) 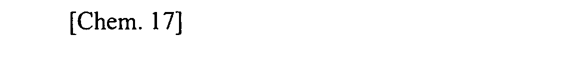 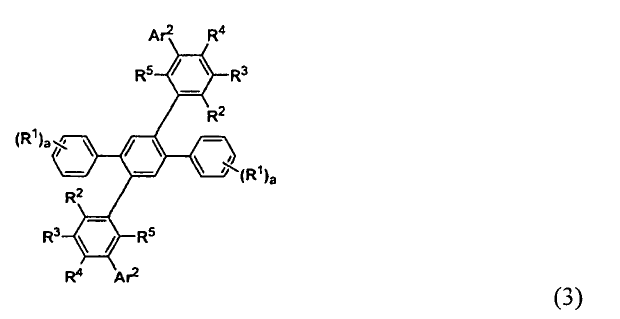 where R¹, R², R³, R⁴, R⁵, Ar² and a are the same as defined in the above formula (3).

### [Feature 16]

The organic electroluminescent device according to Feature 14, wherein the terphenyl derivative is represented by the general formula (4) 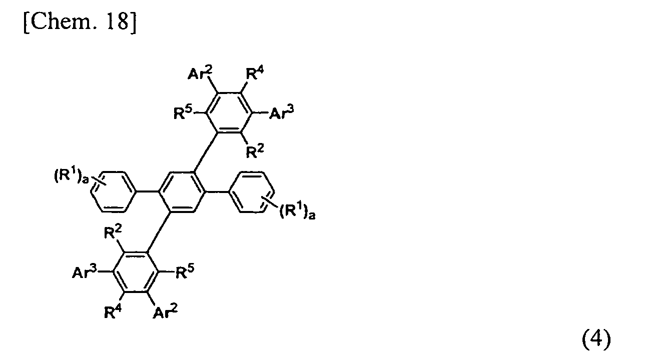 where R¹, R², R³, R⁴, R⁵, *a*, Ar² and Ar³ are the same as defined in the above formula (4).

### [Feature 17]

The organic electroluminescent device according to Feature 14, wherein the terphenyl derivative is represented by the general formula (5) 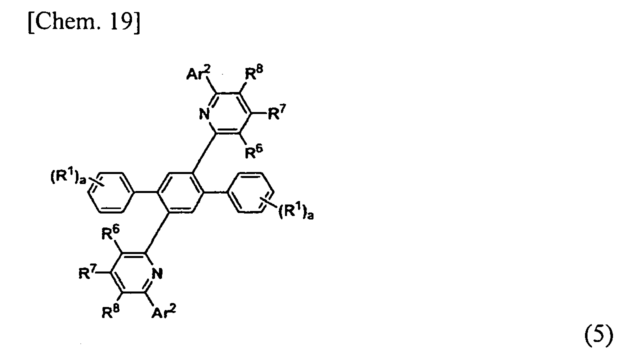 where R¹, *a*, Ar², R⁶, R⁷ and R⁸ are the same as defined in the above formula (4).

### [Feature 18]

The organic electroluminescent device according to Feature 14, wherein the terphenyl derivative is represented by the general formula (6) 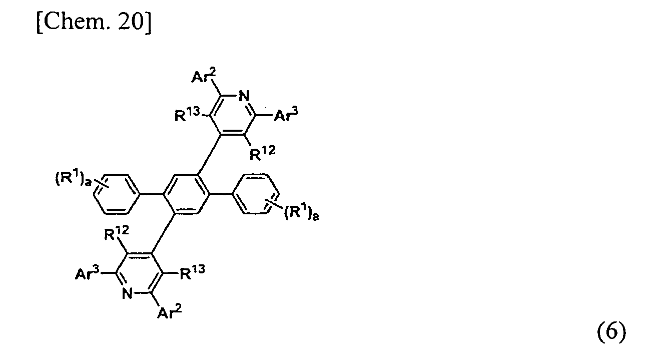 where R¹, R¹², R¹³, Ar², Ar³ and *a* are the same as defined in the above formula (6).

### [Feature 19]

The organic electroluminescent device according to Feature 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (3) 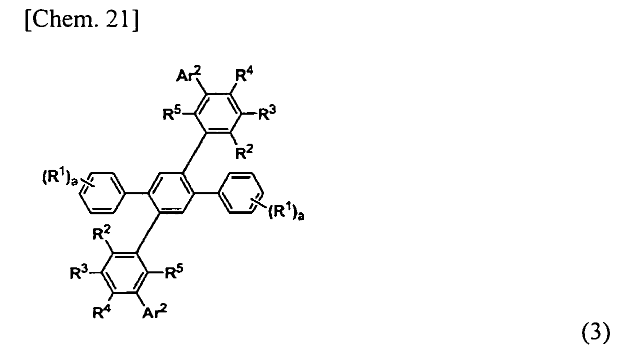 where R¹, *a*, R², R³, R⁴, R⁵ and Ar³ are the same as defined in the above formula (3); and wherein the blue phosphorescent dopant material is FIrpic represented by the following formula

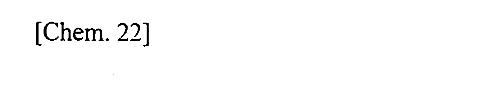

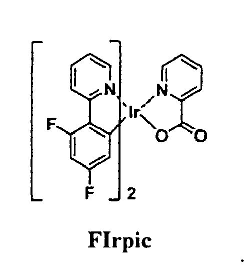

### [Feature 20]

The organic electroluminescent device according to Feature 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (4) 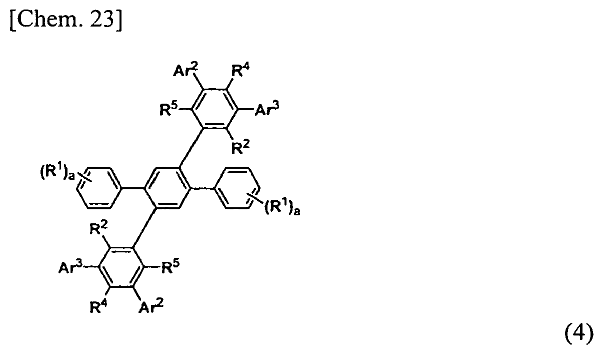 where R¹, R², R³, R⁴, R⁵, *a*, Ar² and Ar³ are the same as defined in the above formula (4); and wherein the blue phosphorescent dopant material is FIrpic represented by the following formula

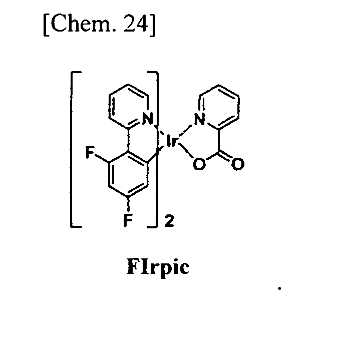

### [Feature 21]

The organic electroluminescent device according to Feature 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (5) 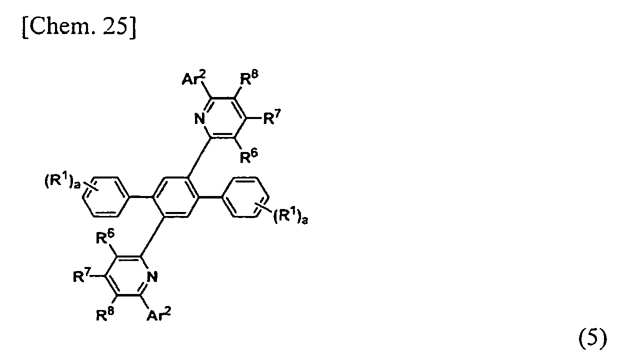 where R¹, *a*, Ar³, R⁶, R⁷ and R⁸ are the same as defined in the above formula (5); and wherein the blue phosphorescent dopant material is FIrpic represented by the following formula

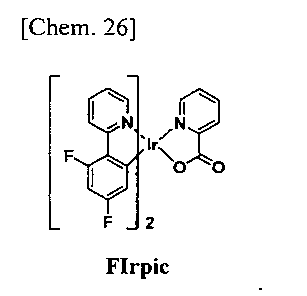

### [Feature 22]

The organic electroluminescent device according to Feature 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (6) 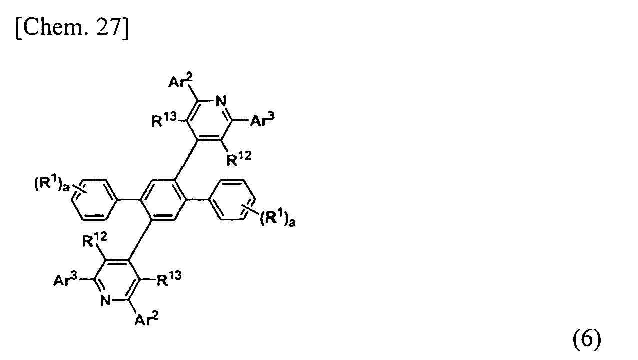 where R¹, *a*, Ar³, R⁶, R⁷ and R⁸ are the same as defined in the above formula (4); and wherein the blue phosphorescent dopant material is FIrpic represented by the following formula

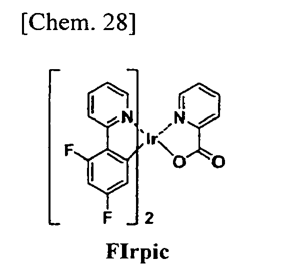

### Brief Description of the Drawings

FIG. 1 is a simplified block diagram showing one example of organic electroluminescent device according to Embodiment 2 of the present invention.

FIG. 2 is a simplified block diagram showing another example of organic electroluminescent device according to Embodiment 2 of the present invention.

### Detailed Description

The present invention provides a novel derivative having an oligophenylene skeleton for use in an organic electroluminescent device. The oligophenylene derivative forms a stable amorphous thin film and has a wide band gap because of its twisted skeleton. It is possible to provide an organic electroluminescent device with a high light emission efficiency with the use of this compound as a material of the organic electroluminescent device. This compound is particularly useful as a host material of a blue phosphorescent organic electroluminescent device so that the organic electroluminescent device can attain a high light emission efficiency.

The oligophenylene derivative of the present invention consists of carbon and hydrogen atoms, or consists of carbon, hydrogen and nitrogen atoms. It is thus advantageous from a production viewpoint that the oligophenylene derivative can be produced at low cost using known compounds as raw materials. Further, the oligophenylene compound can attain material properties such as high heat resistance.

Namely, the organic electroluminescent device according to the present invention has a pair of electrodes and at least one organic light-emitting layer arranged between the electrodes, wherein the organic light-emitting layer contains an oligophenylene derivative represented by the general formula (1) 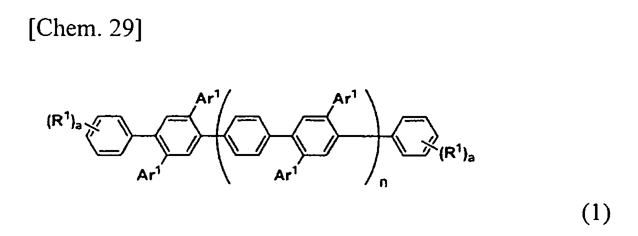 where Ar¹, *n* and R¹ are the same as defined above.
In the formula, Ar¹ each independently represents an oligophenyl group of the formula (1a)

[Chem. 30] - (Ar') _{b} - (Ar") _{c} (1a)

where Ar' each independently represents a divalent to hexavalent aromatic ring which may have a substituent or substituents; and Ar" each independently represents a phenyl group which may have a substituent or substituents. Herein, Ar' and Ar', or Ar' and Ar", are bonded to each other by a single C-C bond between carbon atoms of the respective aromatic rings. A plurality of Ar' and Ar" can be bonded to each other in straight chain form or in branched chain form. Further, Ar' can be either divalent, trivalent, tetravalent, pentavalent or hexavelent. It means that one to five Ar" can be substituted on one Ar'. In any case, Ar¹ as a whole constitutes "a monovalent group (oligophenyl group) having a plurality of six-membered aromatic rings joined together".

Each substituent of Ar' and Ar" can be located at any position on the aromatic ring and is selected from the group consisting of a C₁-C₆ alkyl group, a C₁-C₆ fluoroalkyl group and a halogen atom. Each of Ar' and Ar" may have one to four nitrogen atoms as a heteroatom in the aromatic ring and thus be heterocyclic.

Further, *b* represents an integer of 1 to 4 (i.e. the number of divalent to hexavalent aromatic rings); and *c* represents an integer of 1 to 5 (i.e. the number of phenyl groups). These integers vary depending on the chain length and branching degree of Ar¹.

Furthermore, n is 0 or 1, preferably 0.

Among others, Ar¹ is preferably an oligophenyl group of the formula (1b) corresponding to the case of *c* = 1 or an oligophenyl group of the formula (1c) corresponding to the case of *c* = 2.

[Chem. 31] - (Ar') _{b}- (Ar") (1b)

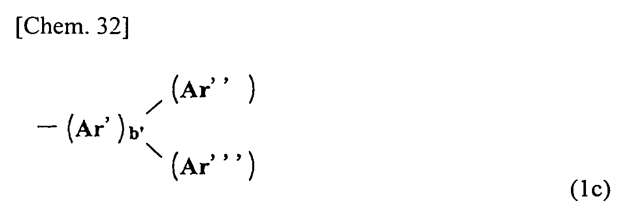

The oligophenyl group of the formula (1b) is an unbranched, straight-chain group. The oligophenyl group of the formula (1c) is a branched group in which Ar" and Ar"' are bonded to one Ar'. In these cases, *b* and *b*' are the same as above. Among others, *b* is preferably 1 or 2 (Ar¹ as a whole constitutes an oligophenyl group having two to four six-membered rings).

It is particularly preferable that the oligophenylene derivative of the present invention satisfies the following conditions: *n* = 0; each of two Ar¹ is either the straight-chain oligophenyl group of the formula (1b) or the branched oligophenyl group of the formula (c); and *b* = 1 for high performance of the electroluminescent device. It is more particularly preferable that the oligophenylene derivative of the present invention satisfies the following conditions: Ar' is the branched oligophenyl group of the formula (1c); and *b* = 1 for high performance and good stability of the electroluminescent device.

Specific examples of Ar¹ in the formula (1) includes biphenyl, terphenyl, tetraphenyl, pentaphenyl, bipyridyl, terpyridyl, tetrapyridyl, pentapyridyl, pyridylphenyl, bipyridylphenyl, pyridyl biphenyl, bipyridyl biphenyl, biphenyl bipyridyl, triphenylphenyl, triphenylpyridyl, tripyridylphenyl, diphenylphenyl, dipyridylphenyl, bis(biphenyl)phenyl, bis(bipyridyl)phenyl, bis(biphenyl)pyridyl and bis(bipyridyl)pyridyl. Among others, biphenyl, bipyridyl, pyridylphenyl, phenylpyridyl, diphenylphenyl and dipyridylphenyl are preferred. Particularly preferred are biphenyl, pyridylphenyl, diphenylphenyl and dipyridylphenyl. Each of Ar¹ may have a substituent or substituents selected from the group consisting of: C₁-C₆ alkyl groups such as methyl, ethyl, n-propyl, i-propyl, n-butyl, i-butyl, s-butyl, t-butyl, pentyl and hexyl; C₁-C₆ fluoroalkyl groups such as trifluoromethyl, pentafluoroethyl, heptafluoropropyl, nonafluorobutyl, undecafluoropentyl and tridecafluorohexyl; and halogen atoms such as fluorine, chlorine, bromine and iodine. Among others, methyl, ethyl, i-propyl, t-butyl, trifluoromethyl, pentafluoroethyl, fluorine, chlorine, bromine and iodine are preferred as the substituent. Particularly preferred are methyl, trifluoromethyl and fluorine.

Each of R¹ is a substituent selected from C₁-C₆ alkyl groups such as methyl, ethyl, n-propyl, i-propyl, n-butyl, i-butyl, s-butyl, t-butyl, pentyl and hexyl; C₁-C₆ fluoroalkyl groups such as trifluoromethyl, pentafluoroethyl, heptafluoropropyl, nonafluorobutyl, undecafluoropentyl and tridecafluorohexyl; and halogen atoms such as fluorine, chlorine, bromine and iodine. Among others, methyl, ethyl, i-propyl, t-butyl, trifluoromethyl and pentafluoroethyl are preferred as the substituent. Particularly preferred are methyl and trifluoromethyl.

The compound of the formula (1) can be, for example, either of terphenyl derivatives of the formulas (3) to (6) 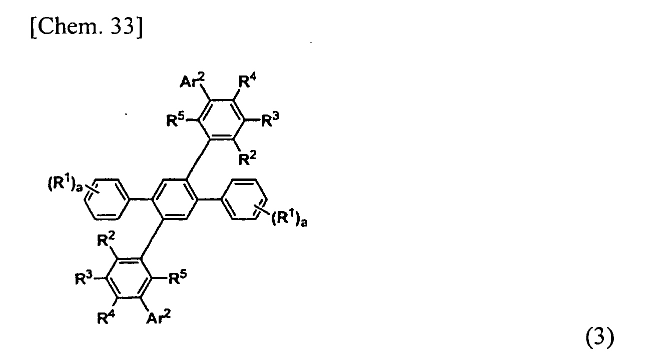 where R¹ is the same as defined in the formula (1); R², R³, R⁴ and R⁵ each independently represent a C₁-C₆ alkyl group, a C₁-C₆ fluoroalkyl group or a halogen atom; Ar² represents a phenyl group which may have a substituent or substituents; each substituent of Ar² can be located at any position on the aromatic ring and is selected from the group consisting of a C₁-C₆ alkyl group, a C₁-C₆ fluoroalkyl group and a halogen atom; Ar² may have one to four nitrogen atoms as a heteroatom in the aromatic ring; and *a* is each independently an integer of 0 to 5; 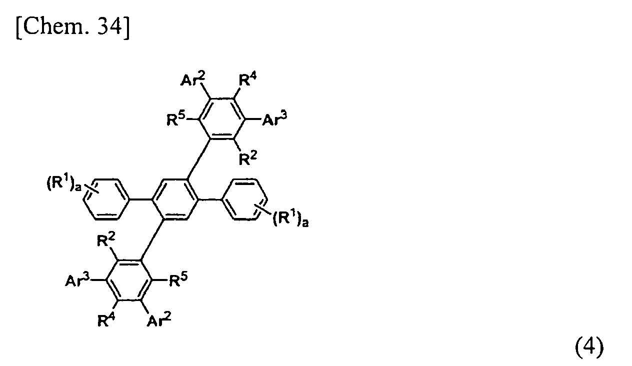 where R¹ and *a* are the same as defined in the formula (1); R², R⁴ and R⁵ each independently represent a C₁-C₆ alkyl group, a C₁-C₆ fluoroalkyl group or a halogen atom; Ar² and Ar³ each independently represent a phenyl group which may have a substituent or substituents; each substituent of Ar² and Ar³ can be located at any position on the aromatic ring and is selected from the group consisting of a C₁-C₆ alkyl group, a C₁-C₆ fluoroalkyl group and a halogen atom; and each of Ar² and Ar³ may have one to four nitrogen atoms as a heteroatom in the aromatic ring; 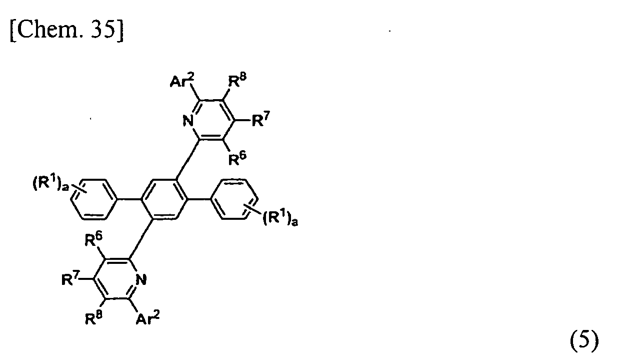 where R¹ is the same as defined in the formula (1); Ar² is the same as defined in the formula (3); and R⁶, R⁷ and R⁸ each independently represent a C₁-C₆ alkyl group, a C₁-C₆ fluoroalkyl group or a halogen atom; 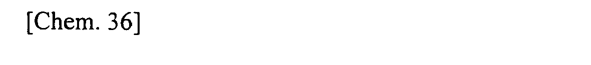 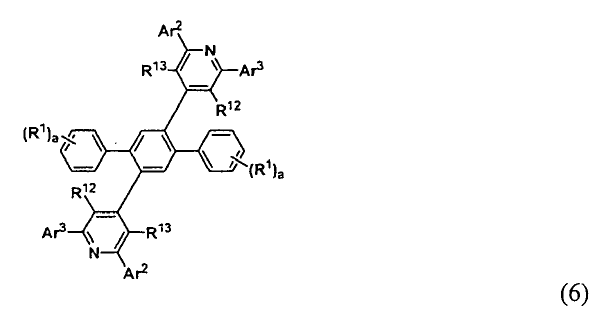 where R¹ and *a* are the same as defined in the formula (1); Ar² and Ar³ are the same as defined in the formula (4); and R¹² and R¹³ each independently represent a C₁-C₆ alkyl group, a C₁-C₆ fluoroalkyl group or a halogen atom.

Specific examples of Ar² and Ar³ in the formulas (3) to (6) includes phenyl, biphenyl, terphenyl, tetraphenyl, pyridyl, bipyridyl, terpyridyl, tetrapyridyl, phenylpyridyl, pyridylphenyl, bipyridylphenyl and biphenylpyridyl. Among others, phenyl and pyridyl are preferred. Each of Ar² and Ar³ may have a substituent or substituents selected from the group consisting of: C₁-C₆ alkyl groups such as methyl, ethyl, n-propyl, i-propyl, n-butyl, i-butyl, s-butyl, t-butyl, pentyl and hexyl; C₁-C₆ fluoroalkyl groups such as trifluoromethyl, pentafluoroethyl, heptafluoropropyl, nonafluorobutyl, undecafluoropentyl and tridecafluorohexyl; and halogen atoms such as fluorine, chlorine, bromine and iodine. Among others, methyl, ethyl, i-propyl, t-butyl, trifluoromethyl, pentafluoroethyl, fluorine, chlorine, bromine and iodine are preferred as the substituent. Particularly preferred are methyl, trifluoromethyl and fluorine.

As each of R² to R⁸, there can be used any of C₁-C₆ alkyl groups such as methyl, ethyl, n-propyl, i-propyl, n-butyl, i-butyl, s-butyl, t-butyl, pentyl and hexyl; C₁-C₆ fluoroalkyl groups such as trifluoromethyl, pentafluoroethyl, heptafluoropropyl, nonafluorobutyl, undecafluoropentyl and tridecafluorohexyl; and halogen atoms such as fluorine, chlorine, bromine and iodine. Among others, methyl, ethyl, i-propyl, t-butyl, trifluoromethyl and pentafluoroethyl are preferred. Particularly preferred are methyl and trifluoromethyl.

The followings are specific examples of the compounds. It is however noted that these compounds are not intended to limit the present invention thereto.

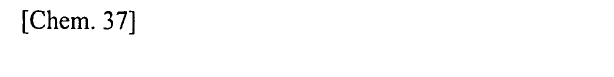

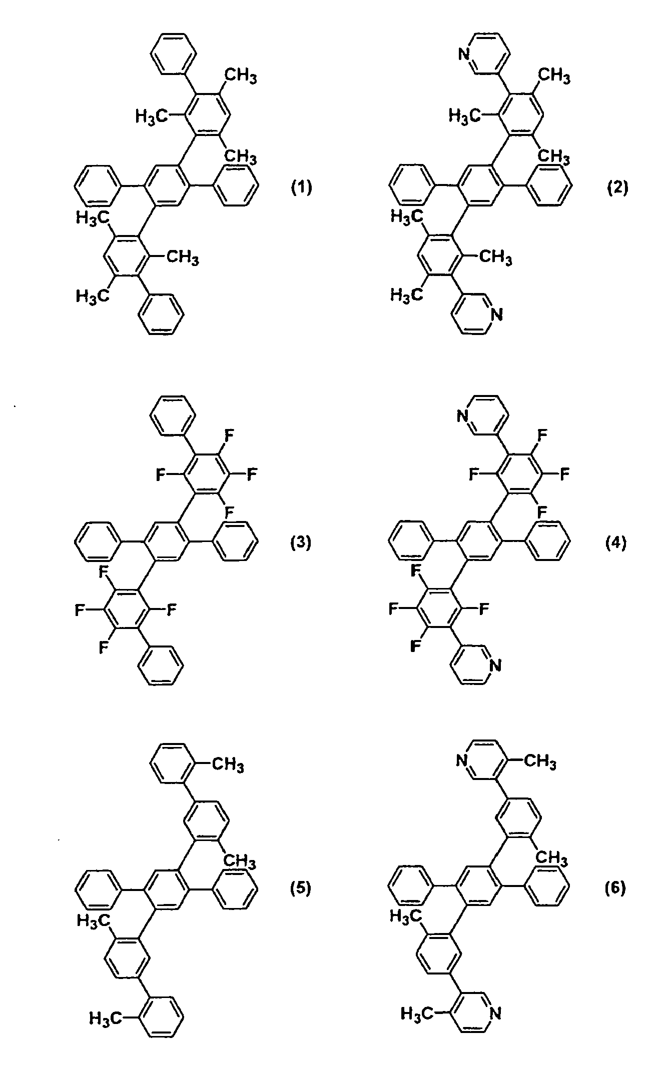

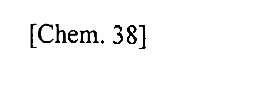

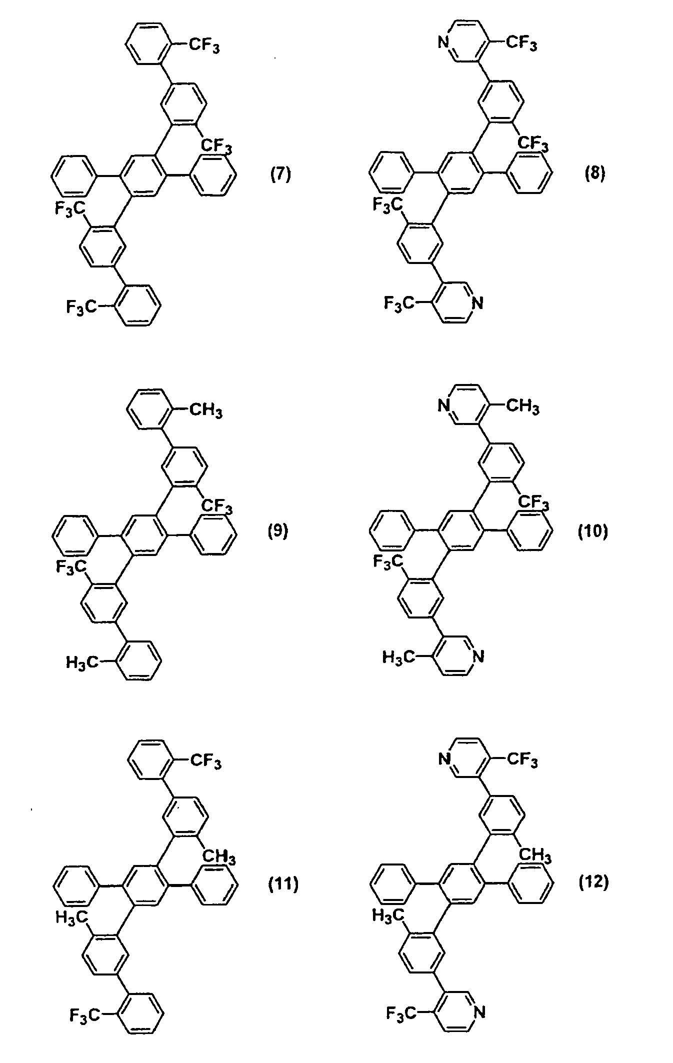

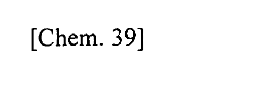

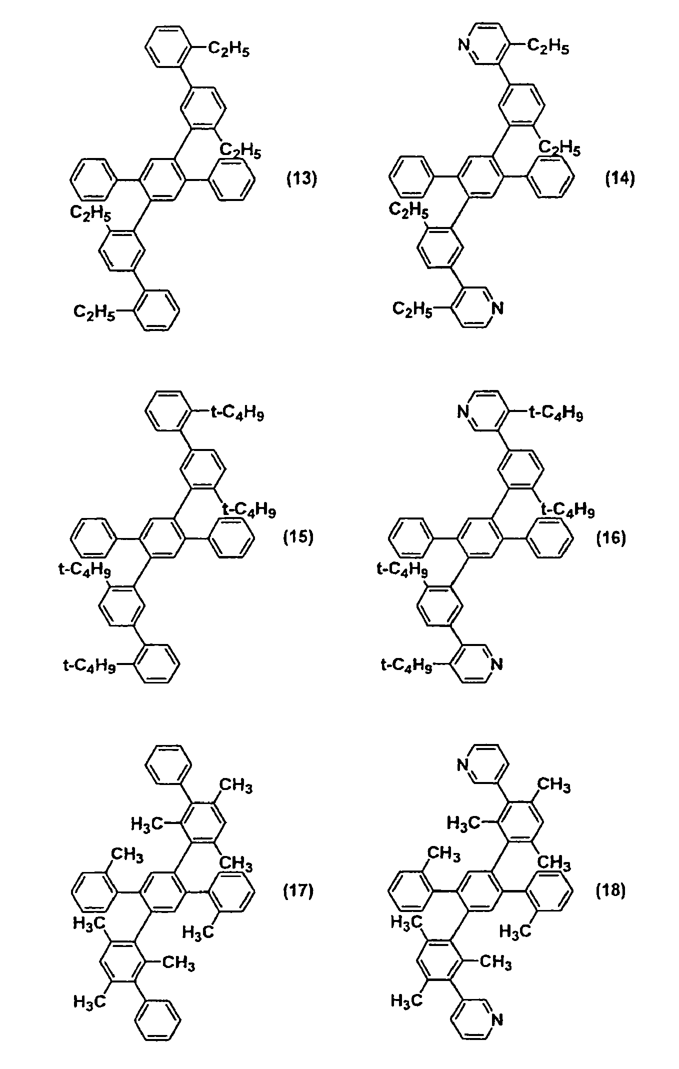

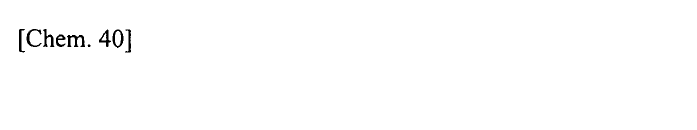

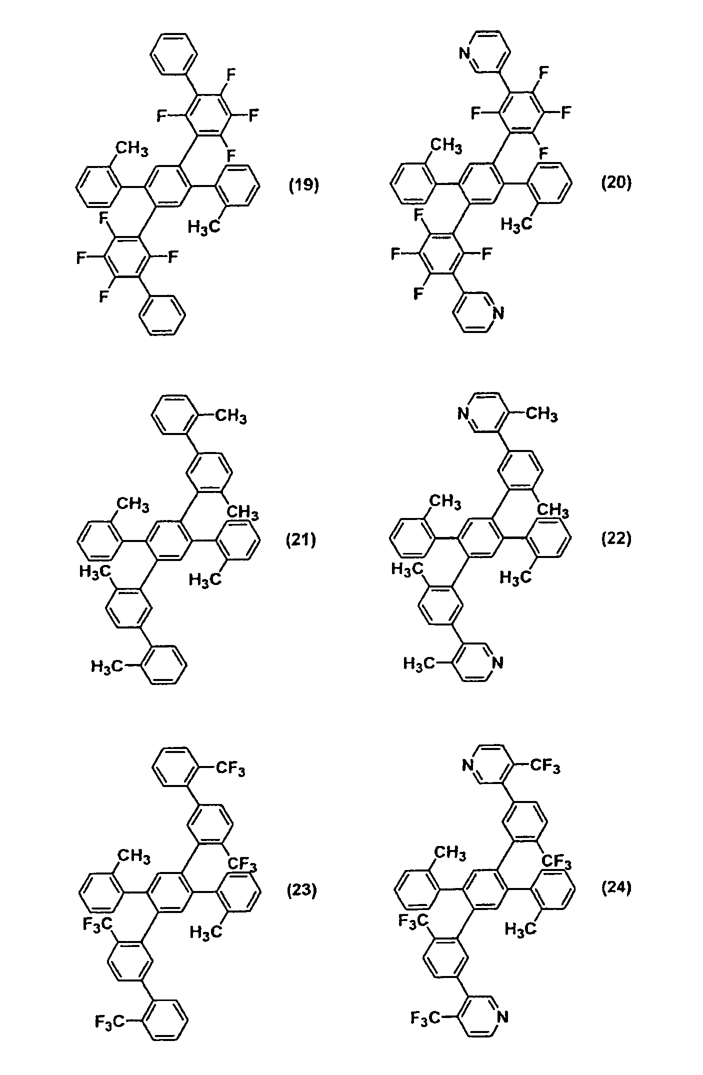

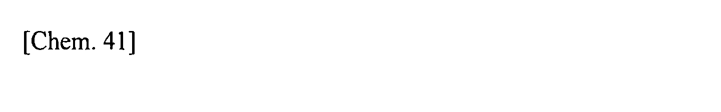

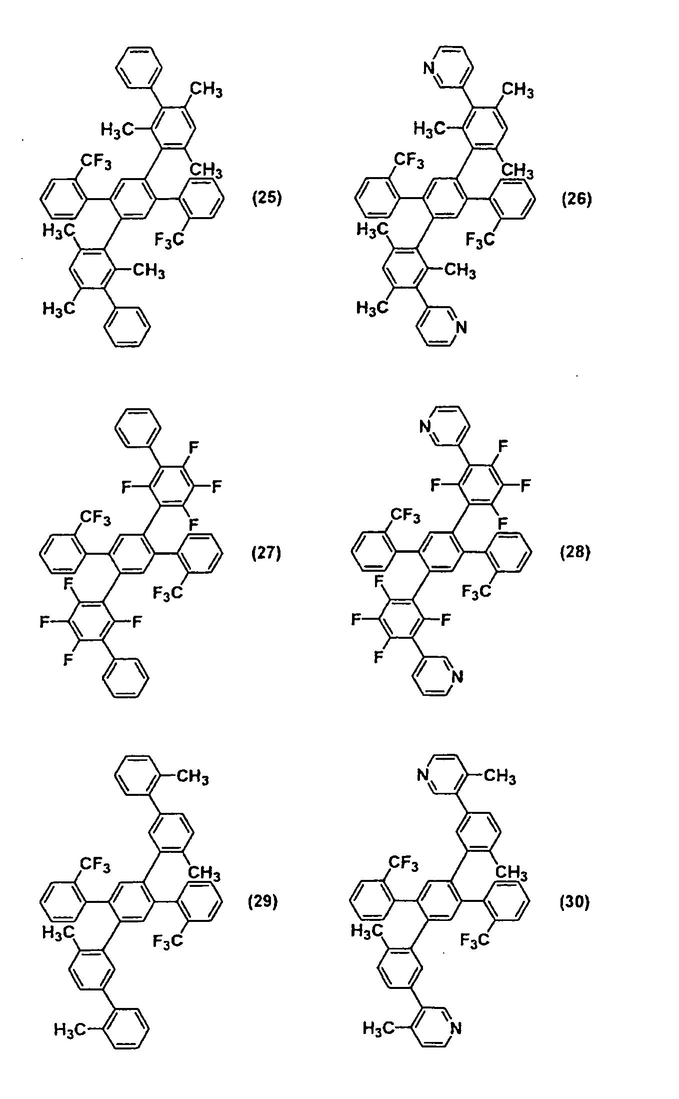

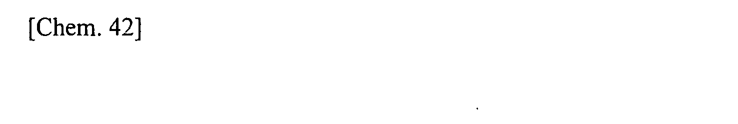

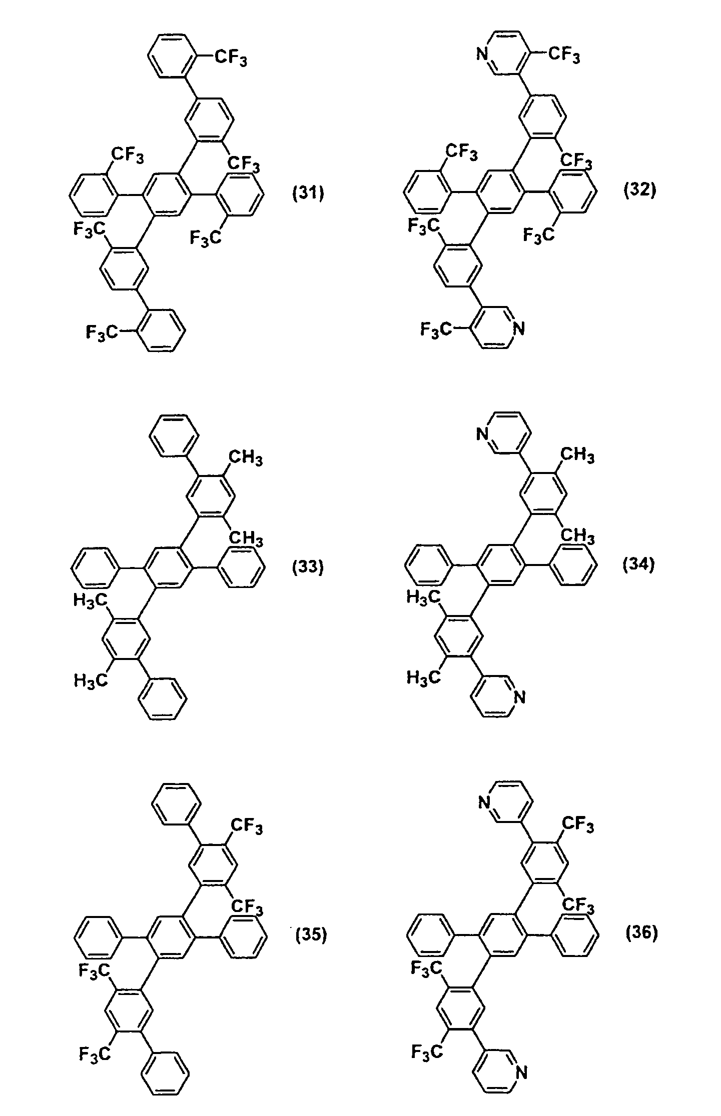

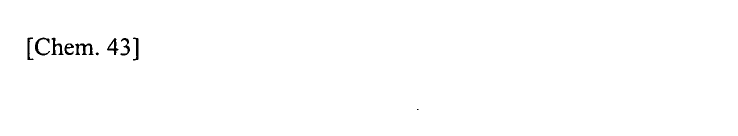

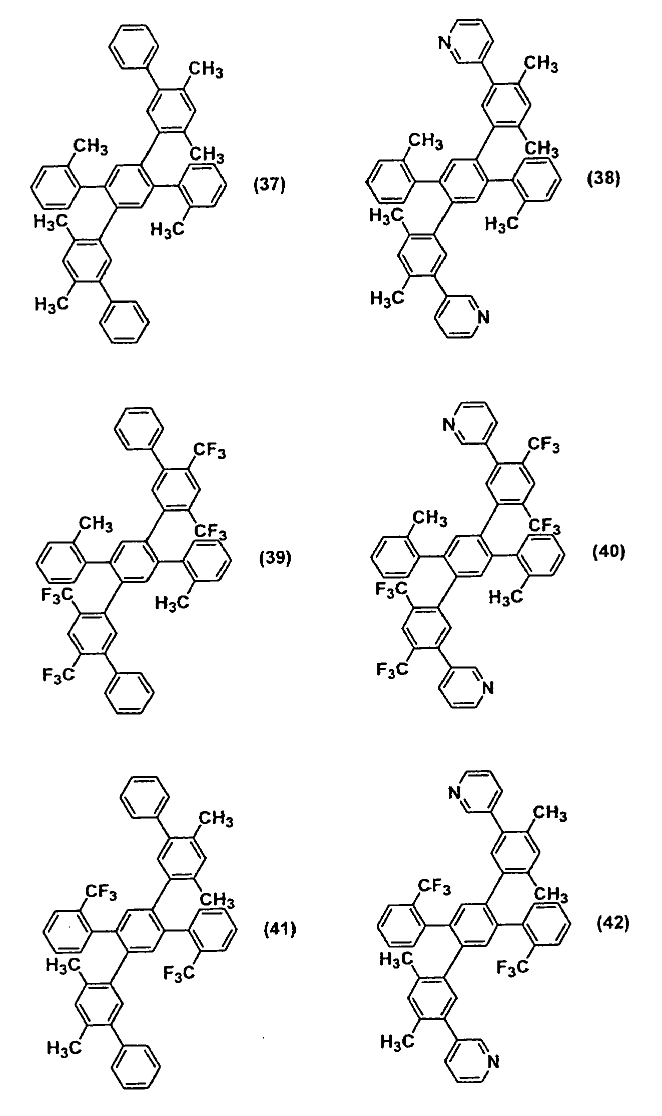

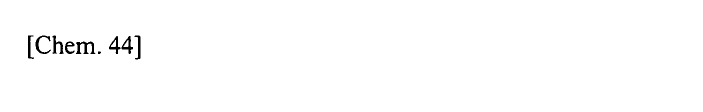

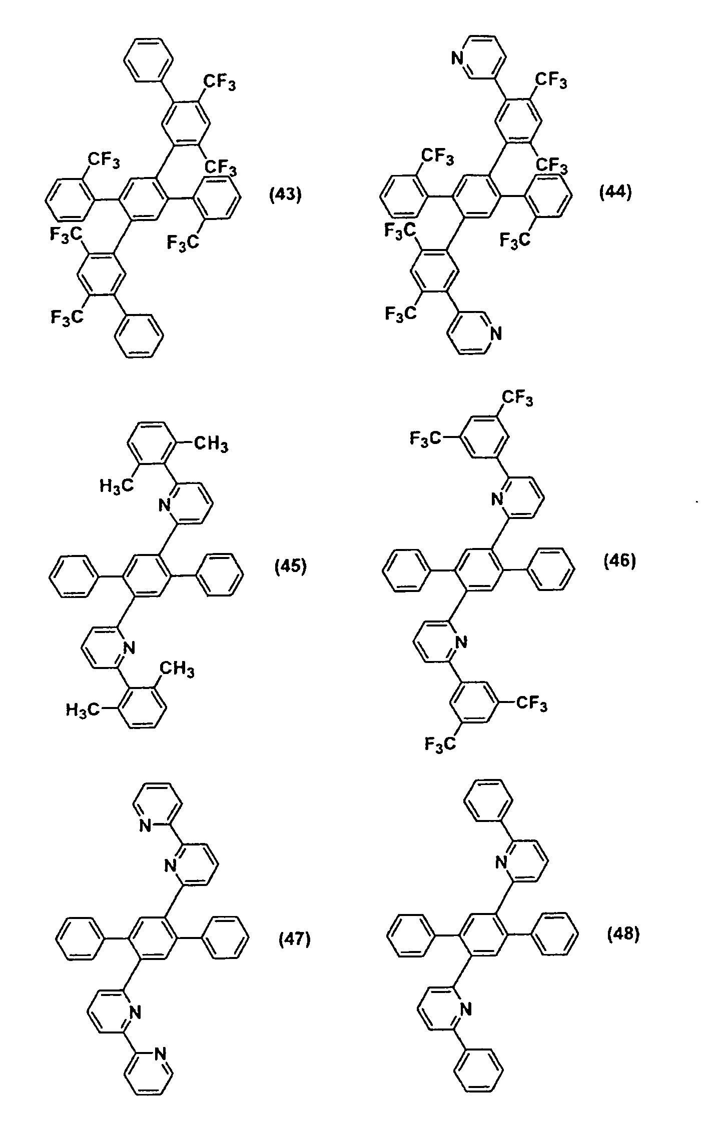

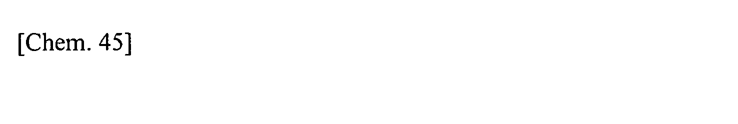

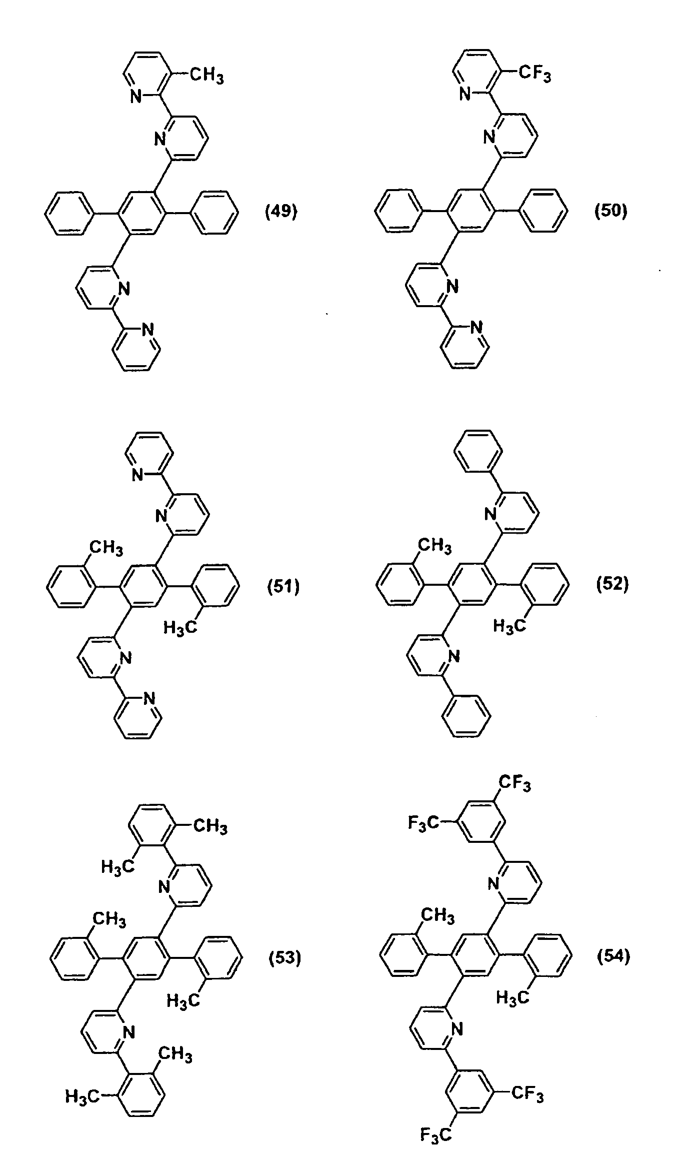

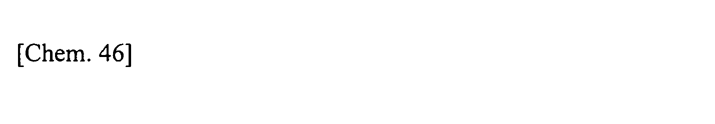

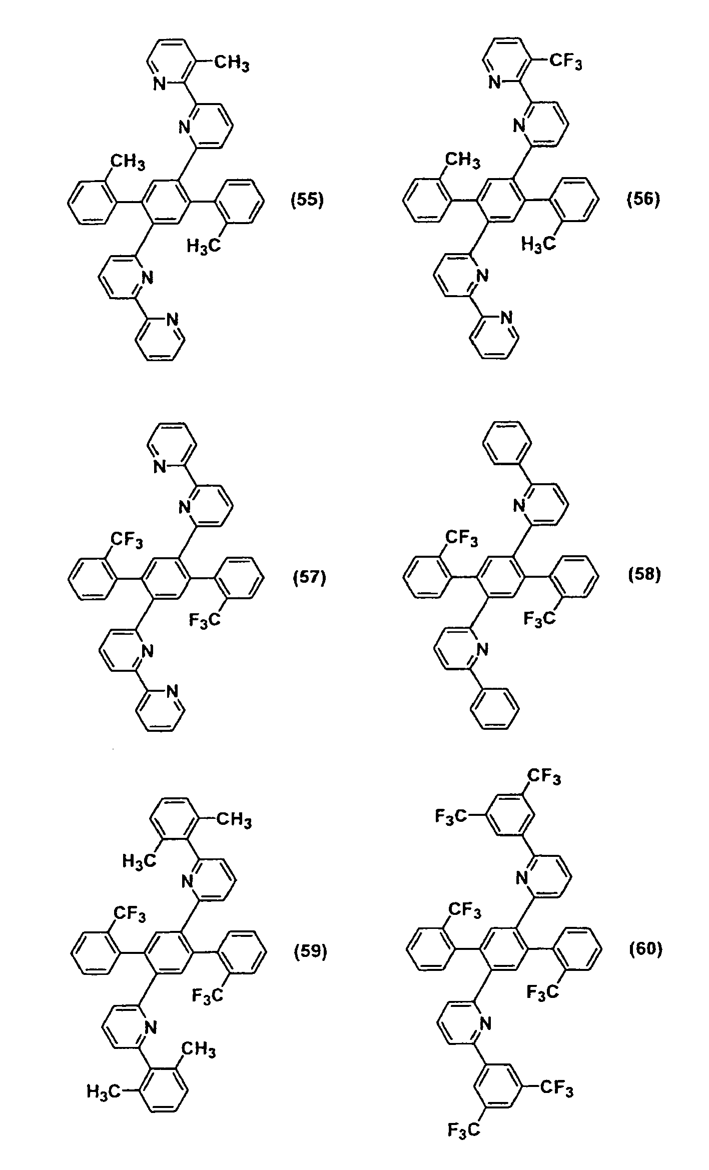

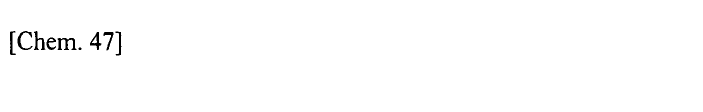

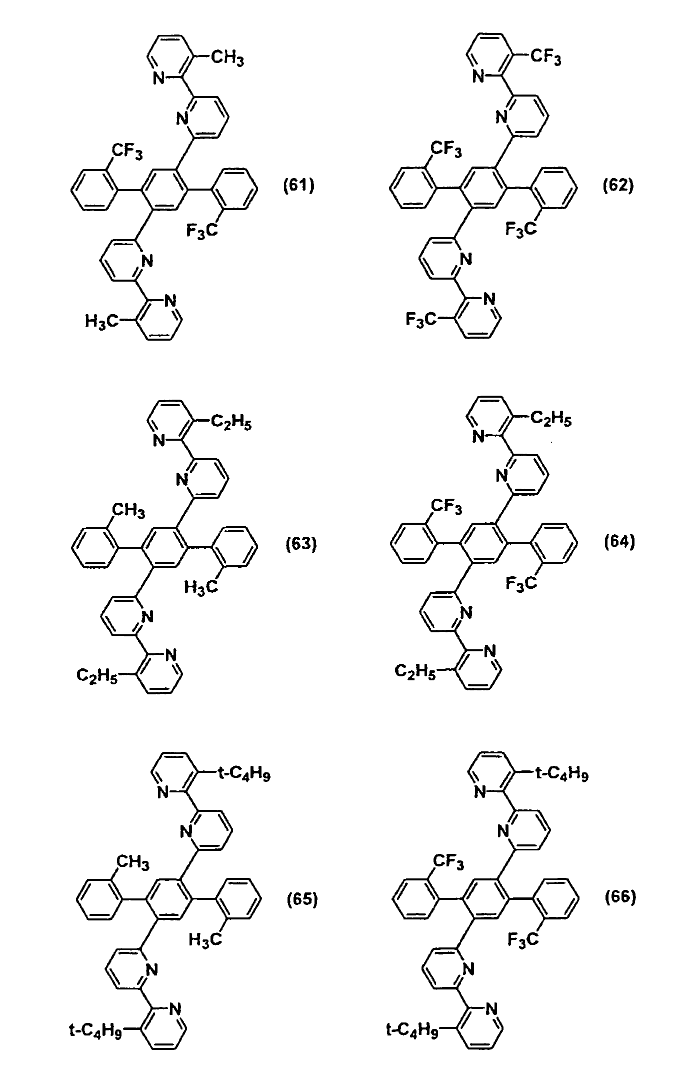

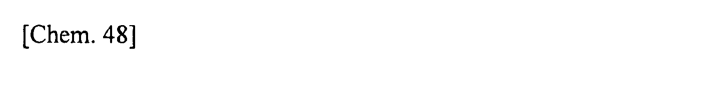

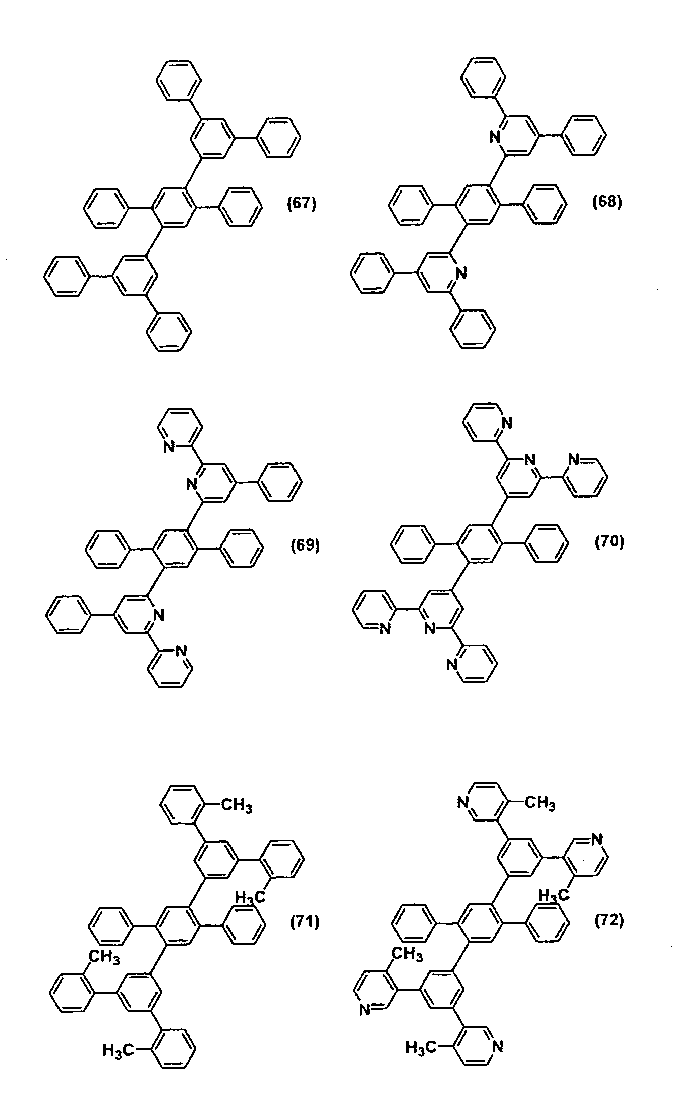

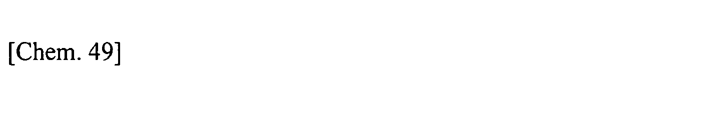

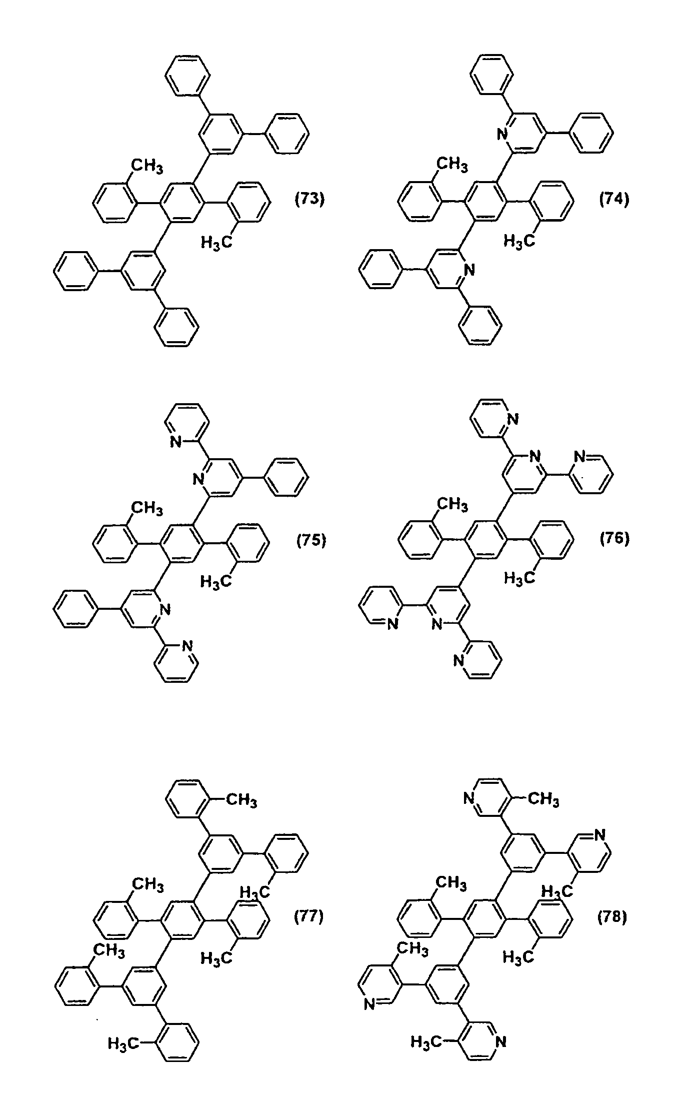

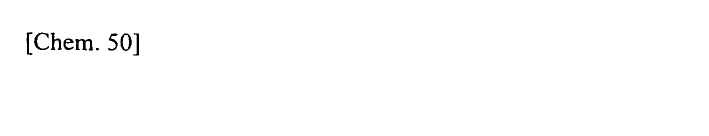

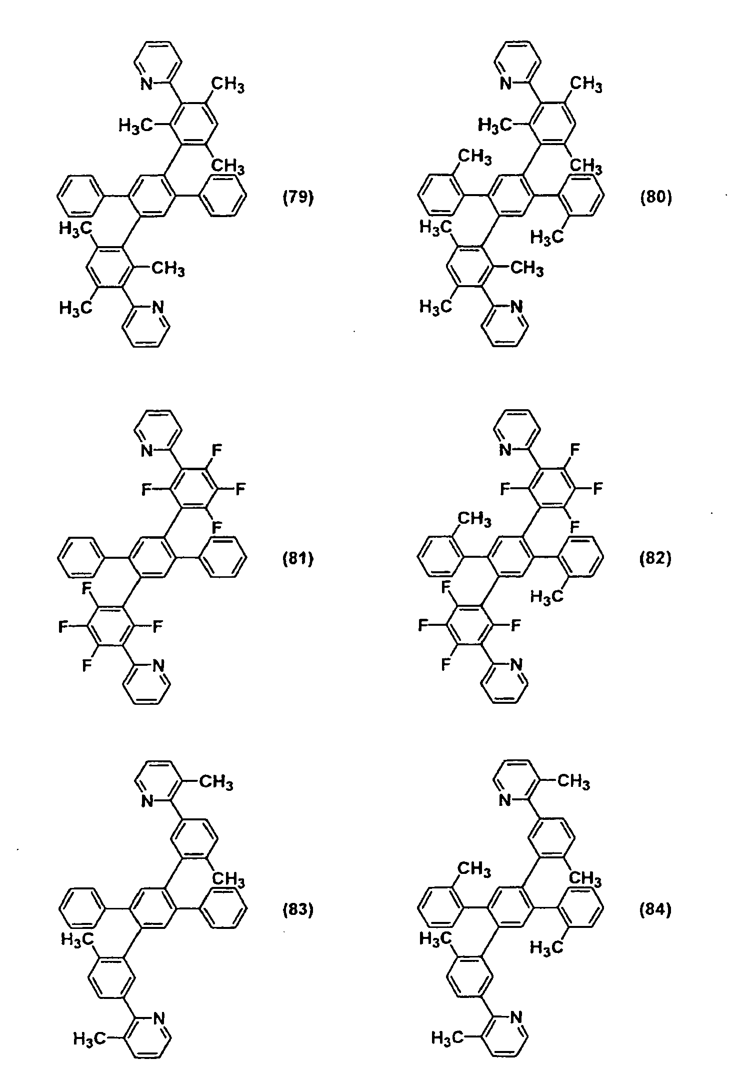

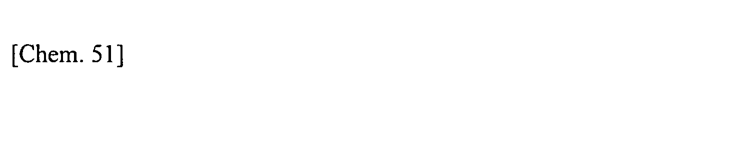

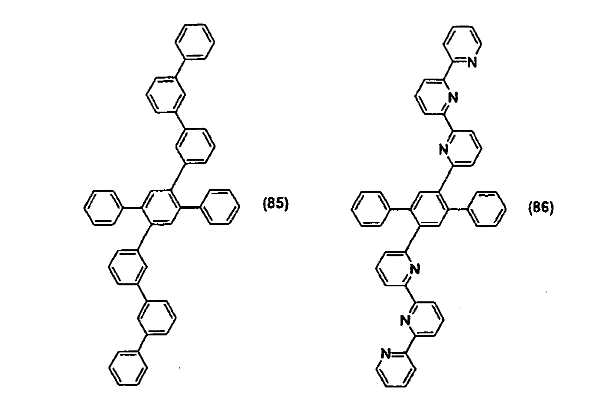

Of the above compounds, preferred are the compounds (1), (2), (4), (6), (21), (22), (71), (72), (77), (78) and (79) each of which shows an absorption edge at a short wavelength of 330 nm or less and has an optical band gap of 3.8 eV or more. It means that these compounds are sufficiently effective in, when used as a host material for a blue phosphorescent dopant (having a band gap on the order of 3 eV), trapping excitation energy of the dopant. This is a result that the host material of the present invention has the synergistic effect of: (1) increasing the flexibility (amorphous nature) of the molecule due to the twisted molecular structure and thereby improving the durability of the light-emitting layer; and (2) weakening the π conjugation of the molecule due to the non-planarity of the molecular structure and thereby increasing the band gap of the material effectively.

### [Embodiment 1: Production of Oligophenylene Derivative]

The production of the oligophenylene derivative of the present invention will be explained below. An oligophenylene derivative of the formula (5) is first prepared by e.g. reacting 1,4-dibromo-2,5-diiodobenzene, which can be obtained by any known process, with a phenyl metal reagent.

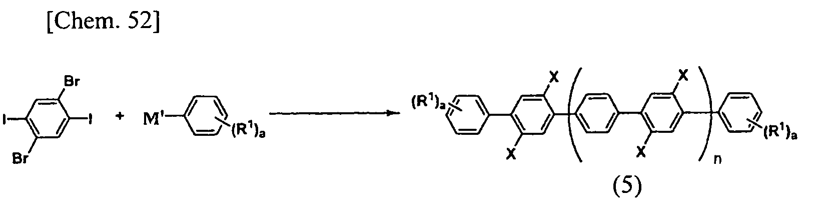

where *n* represents 0 or 1; R¹ each independently represents a C₁-C₆ alkyl group, a C₁-C₆ fluoroalkyl group or a halogen atom; X represents chlorine, bromine or iodine; M' represents a metal group; and *a* is each independently an integer of 0 to 5.

The compound of the formula (5) may alternatively be prepared by using an aryl Grignard reagent as disclosed in J. Org. Chem. 1985, 50, 3104.

Subsequently, Ar¹ is introduced by reacting the compound of the formula (5) with a compound of the formula (6)

[Chem. 53] Ar¹-M" (6)

where Ar¹ represents an oligophenyl group of the formula (1a); and M" represents a metal group or a halogen atom

[Chem. 54] - (Ar')_{b} - (Ar") _{c} (1a)

where Ar' each independently represents a divalent to hexavalent aromatic ring which may have a substituent or substituents; Ar" each independently represents a phenyl group which may have a substituent or substituents; each substituent of Ar' and Ar" can be located at
any position on the aromatic ring and is selected from the group consisting of a C₁-C₆ alkyl group, a C₁-C₆ fluoroalkyl group and a halogen atom; each of Ar' and Ar" may have one to four nitrogen atoms as a heteroatom in the aromatic ring; *b* represents an integer of 1 to 4; *c* represents an integer of 1 to 5; Ar' and Ar' or Ar' and Ar" are bonded to each other by a single C-C bond between carbon atoms of the respective aromatic rings; and *c* number of Ar" can be bonded to Ar' in straight chain form or in branched chain form or directly bonded to Ar'.
At this time, there can be used any of the following reaction processes.

[1] The process of cross-coupling the oligophenylene derivative of the formula (5) with the compound of the formula (6) in the presence of a transition metal catalyst.

[2] The process of converting the oligophenylene derivative of the formula (5) by lithiation with an alkyllithium, cross-coupling with a transition metal catalyst or reaction with a transmetalation reagent such as organomagnesium reagent e.g. alkyl Grignard reagent or alkylmagnesium amide, zinc reagent, tin reagent or borate ester, followed by cross-coupling of the converted derivative with the compound of the formula (6) in the presence of a transition metal catalyst such as a palladium catalyst.

Examples of the alkyllithium usable in the process [2] are n-butyllithium, sec-butyllithium and tert-butyllithium. It is particularly preferable to use tert-butyllithium since the lithiation reaction proceeds with high yield by the use of tert-butyllithium.

Examples of the transmetalation reagent usable in the process [2] are zinc chloride, magnesium chloride, nickel chloride, borate ester, alkyl silyl chloride and alkyl stannyl chloride. Among others, preferred are zinc chloride and borate ester.

Further, there can be used various kinds of transition metal catalysts such as iron catalysts, copper catalysts, cobalt catalysts, nickel catalysts, palladium catalysts, ruthenium catalysts and rhodium catalysts as the cross-coupling reaction catalyst in each of the processes [1] and [2]. Among others, nickel catalysts, palladium catalysts and copper catalysts are preferred. Particularly preferred are palladium catalysts.

Specific examples of the palladium catalysts include palladium bromide, palladium chloride, palladium iodide, palladium cyanide, palladium acetate, palladium trifluoroacetate, palladium acetylacetonate [Pd(acac)₂], diacetatebis(triphenylphosphine)palladium [Pd(OAc)₂(PPh₃)₂], tetrakis(triphenylphosphine)palladium [Pd(PPh₃)₄], dichlorobis(acetonitrile)palladium [Pd(CH₃CN)₂Cl₂], dichlorobis(benzonitrile)palladium [Pd(PhCN)₂Cl₂], dichloro[ 1,2-bis(diphenylphosphino)ethane]palladium [Pd(dppe)Cl₂], dichloro[1,1-bis(diphenylphosphino)ferrocene]palladium [Pd(dppf)Cl₂], dichlorobis(tricyclohexylphosphine)palladium [Pd[P(C₆H₁₁)₃]₂Cl₂], dichlorobis(triphenylphosphine)palladium [Pd(PPh₃)₂Cl₂], tris(dibenzylideneacetone)dipalladium [Pd₂(dba)₃] and bis(dibenzylideneacetone)palladium [Pd(dba)₂]. Among others, phosphine catalysts such as
tetrakis(triphenylphosphine)palladium [Pd(PPh₃)₄], dichloro[1,2-bis(diphenylphosphino)ethane]palladium[Pd(dppe)Cl₂] and dichlorobis(triphenylphosphine)palladium [Pd(PPh₃)₂Cl₂] are preferred.

Other examples of the palladium catalysts are those synthesized by reaction of a palladium complex and a ligand in a reaction system. As the ligand, there can be used triphenylphosphine, trimethylphosphine, triethylphosphine, tris(n-butyl)phosphine, tris(tert-butyl)phosphine, bis(tert-buthyl)methylphosphine, tris(i-propyl)phosphine, tricyclohexylphosphine, tris(o-tolyl)phosphine, tris(2-furyl)phosphine, 2-dicyclohexylphosphinobiphenyl, 2-dicyclohexylphosphino-2'-methylbiphenyl, 2-dicyclohexylphosphino-2',4',6'-triisopropyl-1,1'-biphenyl,
2-dicyclohexylphosphino-2',6'-dimethoxy-1,1'-biphenyl,
2-dicyclohexylphosphino-2'-(N,N'-dimethylamino)biphenyl,
2-diphenylphosphino-2'-(N,N'-dimethylamino)biphenyl,
2-(di-tert-butyl)phosphino-2'-(N,N'dimethylamino)biphenyl,
2-(di-tert-butyl)phosphinobiphenyl, 2-(di-tert-butyl)phosphino-2'-methylbiphenyl, diphenylphosphinoethane, diphenylphosphinopropane, diphenylphosphinobutane, diphenylphosphinoethylene, diphenylphosphinoferrocene, ethylenediamine, N,N',N",N"'-tetramethylethylenediamine, 2,2'-bipyridyl, 1,3-diphenyldihydroimidazoline, 1,3-dimethyldihydroimidazoline, diethyldihydroimidazolylidene, 1,3-bis(2,4,6-trimethylphenyl)dihydroimidazolylidene and 1,3-bis(2,6-diisopropylphenyl)dihydroimidazolylidene. The palladium catalyst coordinated with any of these ligands can be used as the cross-coupling catalyst.

There is no particular restriction on the reaction solvent of the coupling reaction as long as the reaction solvent does not affect the progress of the coupling reaction. Examples of the reaction solvent are: aromatic hydrocarbon solvents such as toluene, xylene and benzene; ester solvents such as methyl acetate, ethyl acetate and butyl acetate; ether solvents such as diethyl ether, tetrahydrofuran, dioxane, dimethoxyethane and diisopropyl ether; amine solvents such as triethylamine and diethylamine; halogenated hydrocarbon solvents such as methyl chloride, chloroform, dichloromethane, dichloroethane and dibromoethane; ketone solvents such as acetone and methyl ethyl ketone; amide solvents such as dimethylformamide and dimethylacetamide; nitrile solvents such as acetonitrile; dimethyl sulfoxide; and water. These solvents can be used solely or in combination of two or more thereof as appropriate. It is preferable that these solvents have previously been subjected to drying and degassing.

### [Embodiment 2: Organic Electroluminescent Device]

Next, an organic electroluminescent device using the oligophenylene derivative of Embodiment 1 of the present invention will be explained below.

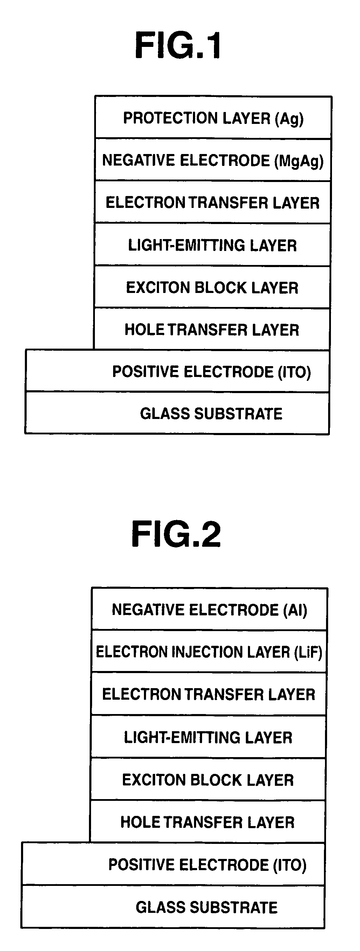

FIG. 1 is a simplified block diagram showing one example of the organic electroluminescent device. The organic electroluminescent device has a transparent substrate of glass, plastic etc. and a transparent electrode formed using ITO (indium tin oxide) etc. on the transparent substrate. The transparent electrode herein functions as a positive electrode. The organic electroluminescent device also has at least one organic layer formed on the transparent electrode.

The at least one organic layer includes at least a light-emitting layer that contains at least the oligophenylene derivative of the present invention.

The organic electroluminescent device of the present invention can adopt various organic layer structures such as a single layer structure consisting of a light-emitting layer and a multilayer structure consisting of a positive hole transfer layer and a light-emitting layer, consisting of a light-emitting layer and an electron transfer layer, or consisting of a positive hole transfer layer, a light-emitting layer or an electron transfer layer depending on the function of the organic compound used and the like. In the present embodiment, the at least one organic layer includes a positive hole transfer layer, an exciton block layer, a light-emitting layer and an electron transfer layer laminated in this order from the transparent electrode side.

The organic electroluminescent device further has a metal electrode formed on the organic layer. The metal electrode herein functions as a negative electrode. The metal electrode can be in the form of a laminate of Mg-Ag alloy electrode and Ag layer etc. (protection layer) as shown in FIG. 1 or a laminate of LiF layer (electron injection layer) and Al electrode as shown in FIG. 2. Although not shown in the drawings, the metal layer can alternatively be formed as a single Al electrode layer or formed of an alloy of Al and alkali metal such as Li or Cs. Further, the organic electroluminescent device may have a positive hole injection layer formed between the transparent electrode and the positive hole transfer layer using copper phthalocyanine (CuPc), starburst amine, vanadium oxide, molybdenum oxide or the like.

The organic electroluminescent device may also have an exciton block layer formed between the positive electrode layer and the light-emitting layer using mCP or the like.

The above-structured organic electroluminescent device of Embodiment 2 uses the oligophenylene derivative of Embodiment 1. The oligophenylene derivative can be used as materials of the positive hole injection layer, the positive hole transfer layer, the exciton block layer, the light-emitting layer, the positive hole block layer, the electron transfer layer and the electron injection layer. It is preferable to use the oligophenylene derivative as the material of the light-emitting layer. Although the oligophenylene derivative can be used solely as the material of the light-emitting layer, it is particularly preferable that the oligophenylene derivative is used as a host material of the light-emitting layer and doped with a certain amount of dopant material (fluorescent material or phosphorescent material) in terms of light emission efficiency, drive power reduction, light color purity improvement etc. Especially, the oligophenylene derivative of the present invention performs an excellent function as a host for a blue phosphorescent material and thus, when used in a blue phosphorescent organic electroluminescent device as the material of the light-emitting layer, can achieve high efficiency and high durability of the organic electroluminescent device.

In other words, the organic electroluminescent device in which the light-emitting layer contains the blue phosphorescent material as the dopant material and the oligophenylene derivative of the present invention as the host material and in which the positive layer, the positive hole transfer layer, the exciton block layer, the light-emitting layer, the electron transfer layer and the negative electrode are laminated in this order as indicated in the drawings is one especially preferred embodiment of the present invention.

It is herein noted that, in the present invention, the term "blue phosphorescence" refers to luminescence derived from a phosphorescent dopant and having a peak wavelength of approximately 400 nm to 480 nm, such as so-called true-blue phosphorescence and light-blue phosphorescence.

The use of the oligophenylene derivative of the present invention is not limited to the above. The oligophenylene derivative of the present invention can suitably be used in a green or red phosphorescent organic electroluminescent device, or used in a blue, green or red fluorescent organic electroluminescent device.

Next, the materials usable together with the oligophenylene derivative of Embodiment 1 in the organic layer of the organic electroluminescent device will be explained below. In the case of using e.g. the oligophenylene derivative of the formula (1) as the host material of the light-emitting layer, there can be used: FIrpic represented by the following formula (12) and FIr6 represented by the following formula (13) as the blue phosphrescent dopant material; Ir(ppy)₃(tris(2-phenylpyridine)mdium (III)) represented by the following formula (14) as the green phosphorescent dopant material; and Ir(pig)₃(tris(2-phenylisoquinoline)iridium (III)) represented by the following formula (15) as the red phosphorescent dopant material

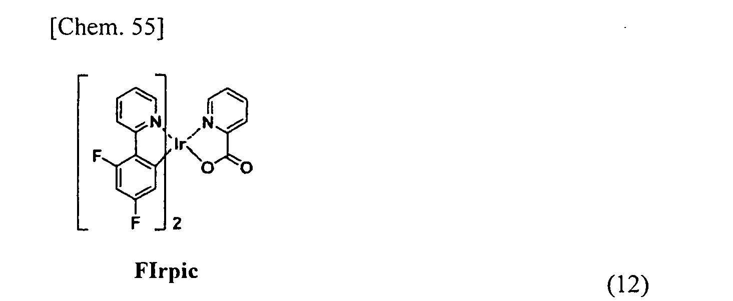

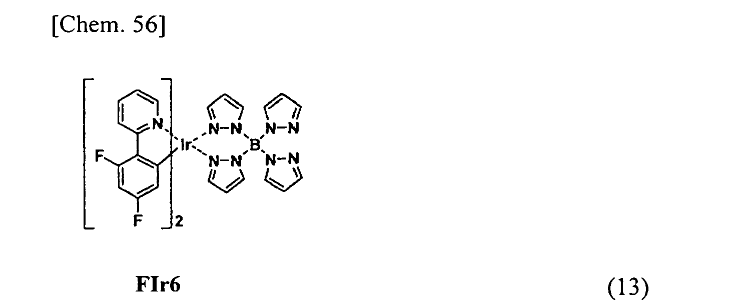

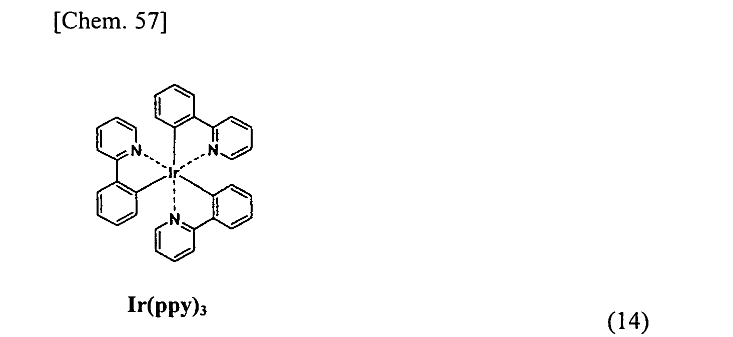

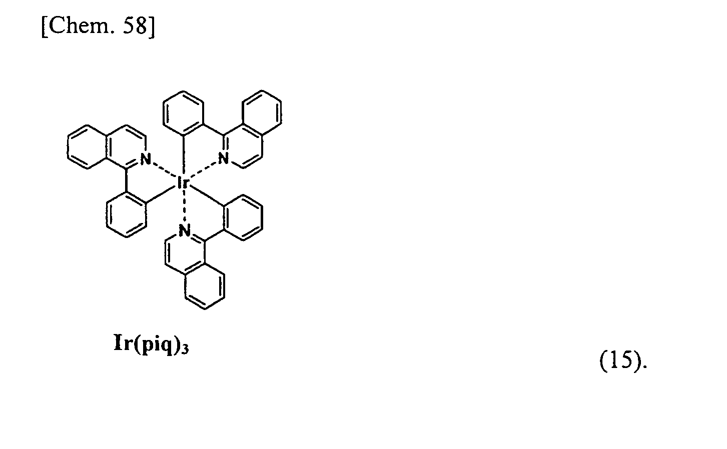

There is no particular restriction on the material of the positive hole transfer layer as long as it has a positive hole transferring function. For example, there can be used α-NPD represented by the following formula (16) and TPTE (trephenylamine tetramer) represented by the following formula (17)

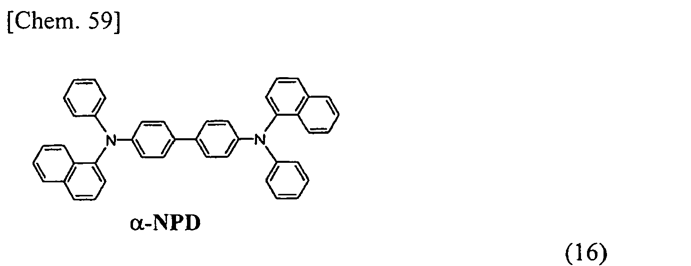

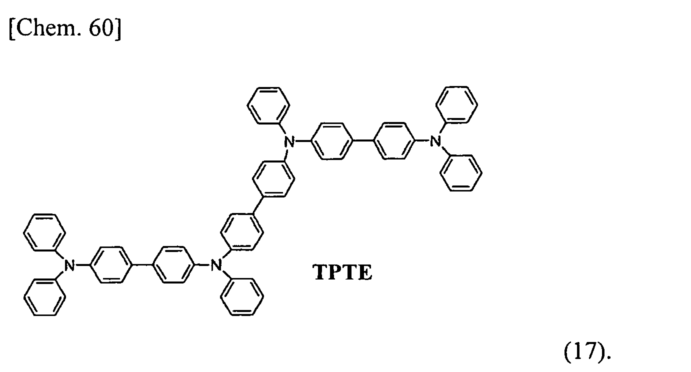

There is also no particular restriction on the material of the electron transfer layer as long as it has an electron transferring function. There can be used alumiquinolinol complex (Alq₃: tris(8-hydroxyquinolinato)aluminum (III)) represented by the following formula (18), bathocuproin (BCP) represented by the following formula (19) and 4,7-diphenyl-1,10-phenanthroline (BPhen) represented by the following formula (20)

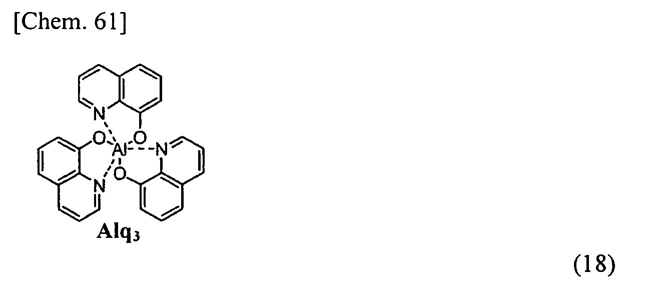

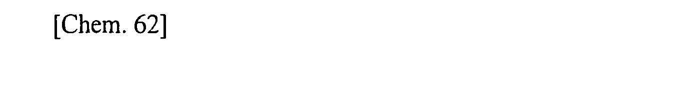

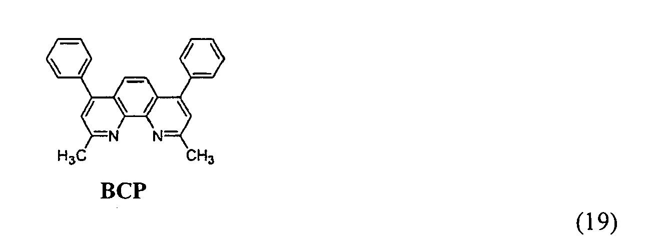

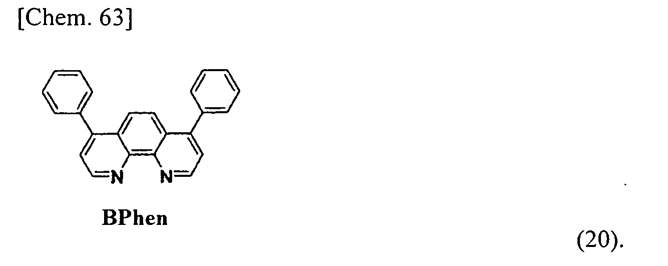

In order to prevent the excitons from flowing out of the light-emitting layer into the positive hole transfer layer, it is preferable that the exciton block layer is formed between the light-emitting layer and the positive hole transfer layer. As the material of the exciton block layer, there can be used mCP represented by the following formula (22)

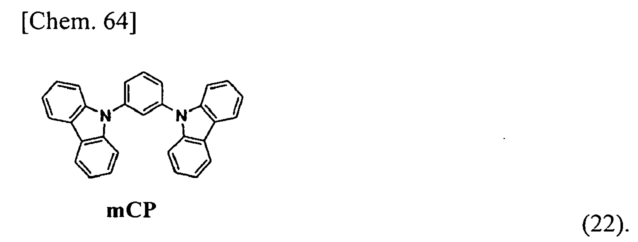

Next, the light emission principle of the organic electroluminescent device of Embodiment 2, in which the oligophenylene derivative of Embodiment 1 is used as the host material for phosphorescent light-emitting material, will be explained below.

Positive holes and electrons are injected from the transparent electrode and metal electrode, which function as the positive and negative electrodes, respectively, to the organic layer. The positive holes are transferred through the positive hole transfer layer, whereas the electrons are transferred through the electron transfer layer and the positive hole block layer. Then, the positive holes and electrons reach the organic layer and get recombined together. The oligophenylene derivative, which serves as the host material of the light-emitting layer, is brought into excited states by recombination of the positive holes and electrons. As mentioned before, singlet and triplet excited states contribute 25% and 75% of the excited states, respectively. The excitation energy of such a singlet/triplet excited host material is transferred to the dopant material so that the dopant material is brought into singlet and triplet excited states. The singlet excited state of the dopant material is further converted to the triplet excited state. In the end, the dopant material principally emits phosphorescence from the triplet excited state. In this way, almost all of the energy of the generated excited states is used as luminescent energy. Alternatively, the electrons and positive holes may be recombined directly at the dopant material in the host material so as to form triplet excitons with an efficiency of 100%.

It is possible in the present embodiment to obtain each of blue phosphorescent emission, green phosphorescent emission and red phosphorescent emission with high color purity and high efficiency depending on the phosphorescent dopant material by using the oligophenylene derivative as the host material. It is also possible to adjust the band gap width (absorption edge value) of the oligophenylene derivative and thereby design the host material most suitable for the blue, green or red phosphorescent dopant by changing the substituent group on the skeleton of the oligophenylene derivative. The oligophenylene derivative of the formula (1) can particularly be used as the host material for the blue phosphorescent dopant such as FIrpic or FIr6, which corresponds to a preferred embodiment that takes full advantage of the excellent properties of the host material.

Among others, an especially preferred embodiment of the organic electroluminescent device that takes full advantage of the properties of the oligophenylene derivative of the present invention is a blue phosphorescent organic electroluminescent device including a pair of electrodes, at least one organic light-emitting layer arranged between the electrodes, a positive hole transfer layer, an electron block layer or an exciton block layer, and an electron transfer layer, wherein the organic light-emitting layer contains a host material and a blue phosphorescent dopant material; the host material is either one of: 1,4-bis(2',4',6'-trimethyl-3'-(3"-pyridyl)phenyl)-2,5-diphenylbenzene (PTP-PyMS) represented by the following formula; 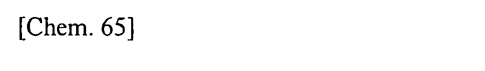 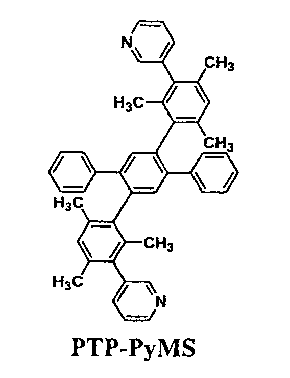 1,4-bis(2',4',6'-trimethyl-3'-phenylphenyl)-2,5-diphenylbenzene (PTP-PMS) represented by the following formula; 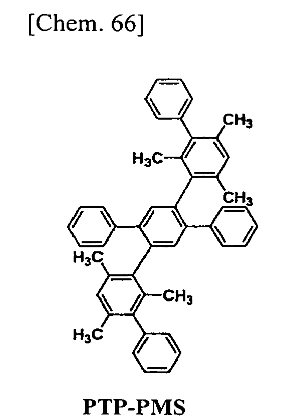 1,4-bis(2'-pyridyl-3'-(2",6"-dimethylphenyl))-2,5-diphenylbenzene (PTP-DMPPy) represented by the following formula; 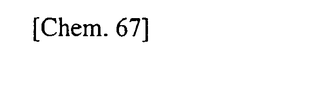 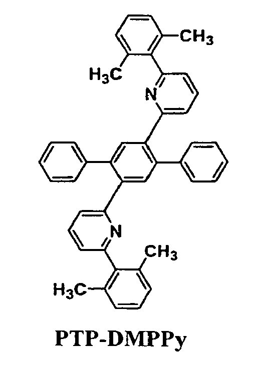 1,4-bis((2',4',5',6'-tetrafluoro-3'-(3"-pyridyl))phenyl)-2,5-diphenylbenzene (PTP-Py4FP) represented by the following formula; 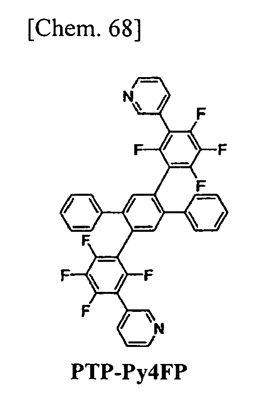 1,4-bis((6'-methyl-3'-(2"-methyl)phenyl)phenyl)-2,5-diphenylbenzene (PTP-MPMP) represented by the following formula; 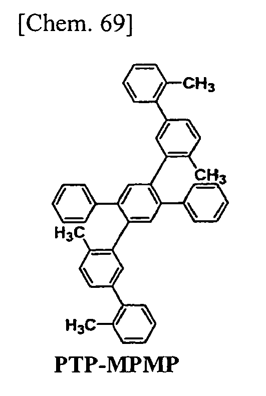 1,4-bis((6'-methyl-3'-(6"-methyl)-3"-pyridyl)phenyl)-2,5-diphenylbenzene
(PTP-MPyMP) represented by the following formula; 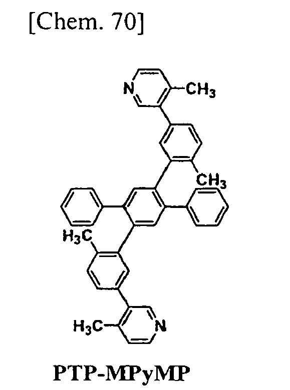 1,4-bis((6'-methyl-3'-(2"-methyl)phenyl)phenyl)2,5-bis(2'-methylphenyl)benzene (CH3-PTP-MPMP) represented by the following formula; 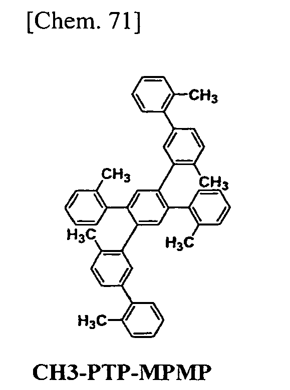 1,4-bis(6'-methyl-3'-(6"-methyl)-3"-pyridyl)phenyl)2,5-bis(2'methylphenyl)benzene (CH3-PTP-MPyMP) represented by the following formula; 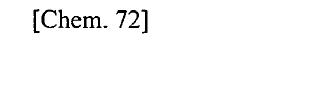 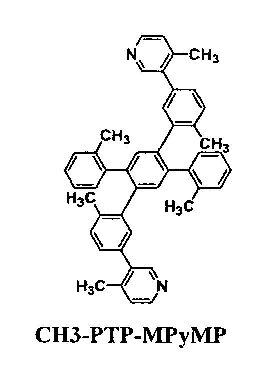 1,4-bis(2',4',6'-trimethyl-3'-(2"-pyridyl)phenyl)-2,5-diphenylbenzen (PTP-2PyMS) represented by the following formula; 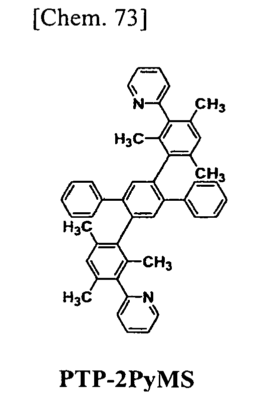 1,4-bis(3',5'-bis((2"-methyl)-phenyl)phenyl)-2,5-diphenylbenzene (PTP-BMPP) represented by the following formula; 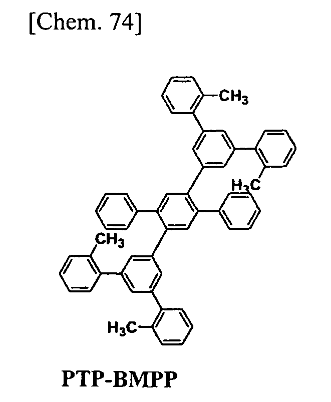 1,4-bis(3',5'-bis((2"-methyl)-phenyl)phenyl)-2,5-bis(2'-methylphenyl)benzene
(CH3-PTP-BMPP) represented by the following formula; 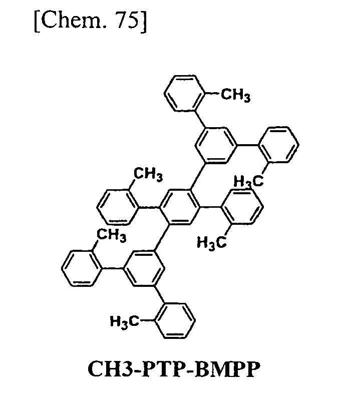 1,4-bis(3',5'-bis((6"-methyl)-3"-pyridyl)phenyl)-2,5-diphenylbenzene (PTP-BMPyP) represented by the following formula; 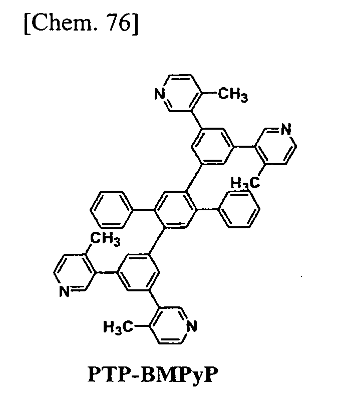 1,4-bis(3',5'-bis((6"-methyl)-3"-pyridyl)phenyl-2,5-bis(2'-methylphenyl)benzene (CH3-PTP-BMPyP) represented by the following formula; 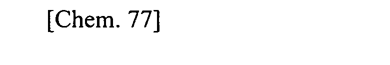 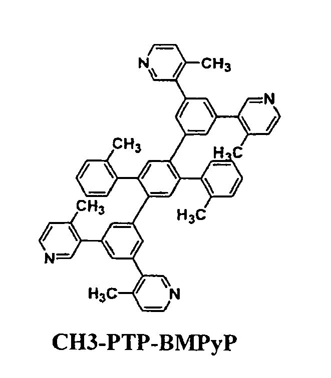 and the blue phosphorescent dopant material is FIrpic represented by the following formula

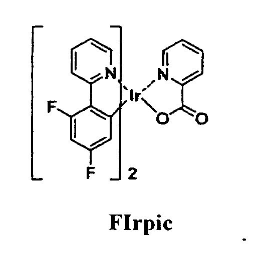

It is more preferable to use, as the host material, PTP-PyMS, PTP-PMS, PTP-MPyMP, CH3-PTP-MPyMP, PTP-2PyMS, PTP-BMPP, CH3-PRP-BMPP, PTP-BMPyP or CH3-PTP-BMPyP.

The use of the oligophenylene derivative of Embodiment 1 is not limited to the organic electroluminescent device. The oligophenylene derivative of Embodiment 1 can suitably be put to a wide range of uses such as: displays or backlights of display devices, computers, televisions, mobile phones, digital cameras, PDA, car navigation systems and the like; illumination lights, interior decorations, signs, traffic lights, billboards and the like; recording/reading light sources for CD, DVD and the like; light sources of copiers, scanners and the like; dyes for use in recording layers of recordable optical discs e.g. CD-R and DVD-R; laser dyes; sensitizing dyes; and fluorescent drugs for medical diagnosis

Further, the organic electroluminescent device of Embodiment 2 can suitably be used for: displays of display devices, computers, televisions, mobile phones, digital cameras, PDA, car navigation systems and the like; light sources such as backlights; illumination lights; interior decorations; signs; traffic lights; billboards; and the like.

### [Examples]

The present invention will be described in more detail below by way of the following examples. It is however noted that these examples are not intended to limit the present invention thereto.

### Example 1

### Production of 1,4-bis(2',4',6'-trimethyl-3'-(3" -pyridyl)phenyl)-2,5-diphenylbenzene (PTP-PyMS)

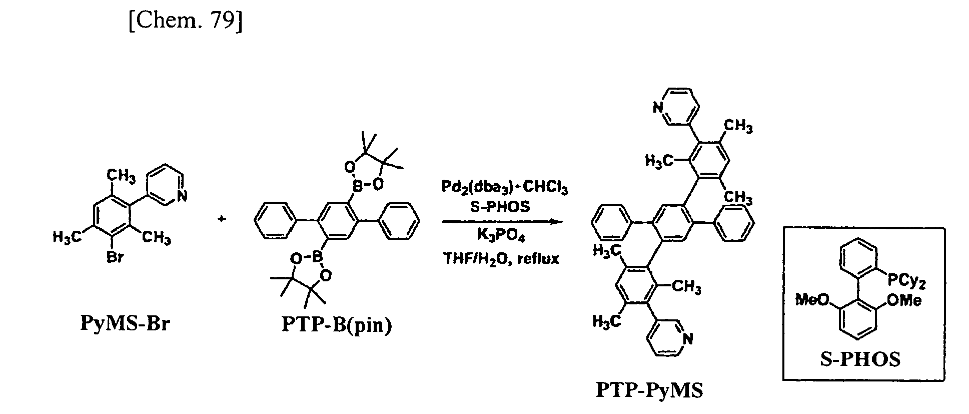

A recovery flask was charged with PyMS-Br (1.09 g, 3.95 mmol), PTP-B(pin) (785 mg, 1.63 mmol), Pd₂(dba)₃·CHCl₃ (45.1 mg, 0.044 mmol), S-PHOS (69.5 mg, 0.169 mmol) and K₃PO₄ (701 mg, 3.30 mmol), followed by adding thereto THF (10 mL) and H₂O (5 mL). The resulting mixture was heat-refluxed. After the completion of the reaction, the mixture was subjected to separation with dichloromethane. The separated solution was dried with Na₂SO₄ and passed through a silica gel. A crude product of PTP-PyMS was obtained by distilling the solvent from the solution under reduced pressure. The crude product was purified by preparative GPC (solvent: chloroform), thereby yielding the target compound in white solid form (712 mg, 70%). ¹H NMR (400 MHz, CDCl₃): ≡ 8.56 (s, 2H), 8.44 (s, 1H), 8.12 (s, 1H), 7.51 (d, J_{HH} = 7.6 Hz, 1H), 7.38-7.30 (m, 4H), 7.22-7.10 (m, 11 H), 6.99 (s, 2H), 2.09 (s, 6H), 1.98 (s, 6H), 1.64 (s, 6H). EI MS m/z 620.0 (M⁺).
As a result of property measurements, it has been verified that the produced compound had a glass transition temperature of 110°C and a triplet excited state energy of 2.7 eV and showed significantly superior properties to those of CBP of Comparative Example 1.

### Example 2

### Production of 1,4-bis(2',4',6'-trimethyl-3'-phenylphenyl-2,5-diphenylbenzene (PTP-PMS)

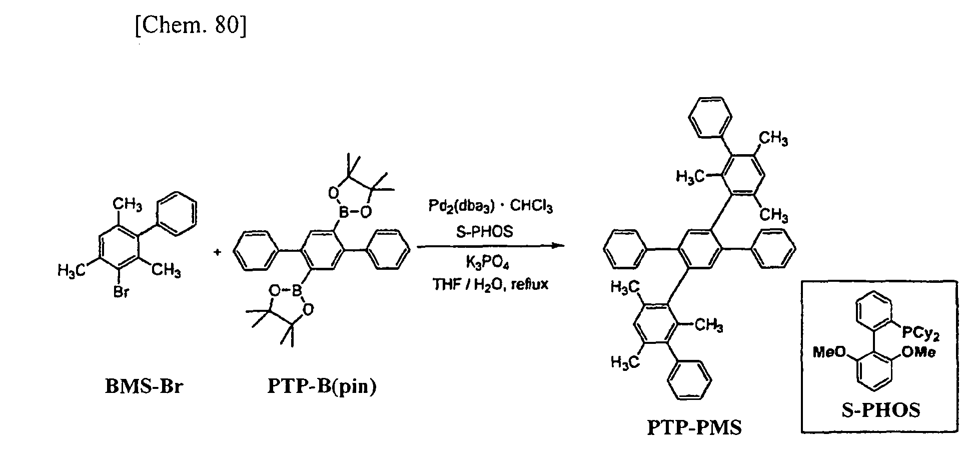

A recovery flask was charged with PMS-Br (1.16 g, 4.20 mmol), PTP-B(pin) (844 mg, 1.75 mmol), Pd₂(dba)₃·CHCl₃ (45.5 mg, 0.044 mmol), S-PHOS (71.8 mg, 0.175 mmol) and K₃PO₄ (743 mg, 3.50 mmol), followed by adding thereto THL (10 mL) and H₂O (5 mL). The resulting mixture was heat-refluxed. After the completion of the reaction, the mixture was subjected to separation with chloroform. The separated solution was dried with MgSO₄ and passed through a silica gel. A crude product of PTP-PMS was obtained by distilling the solvent from the solution under reduced pressure. The crude product was purified by preparative GPC (solvent: chloroform), thereby yielding the target compound in white solid form (720 mg, 67%). ¹H NMR (400 MHz, CDCl₃): ≡ 8.58-8.54 (m, 2H), 8.44 (s, 1H), 8.11 (d, J_{HH} = 2.0 Hz, 1H), 7.51 (d, J_{HH} = 7.2 Hz, 1H), 7.38-7.32 (m, 4H), 7.21-7.13 (m, 11H), 6.99 (s, 2H), 2.10-2.09 (m, 6H), 1.98 (s, 6H), 1.64-1.63 (m, 2H). EI MS m/z 618.6 (M⁺).
As a result of property measurements, it has been verified that the produced compound had a glass transition temperature of 100°C and a triplet excited state energy of 2.7 eV and showed significantly superior properties to those of CBP of Comparative Example 1.

### Example 3

### Production of 1,4-bis(2'-pyridyl-3'-(2",6"-dimethylphenyl))-2,5-diphenylbenzene (PTP-DMPPy)

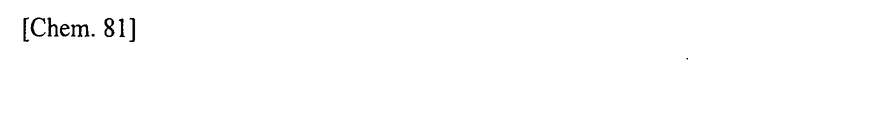

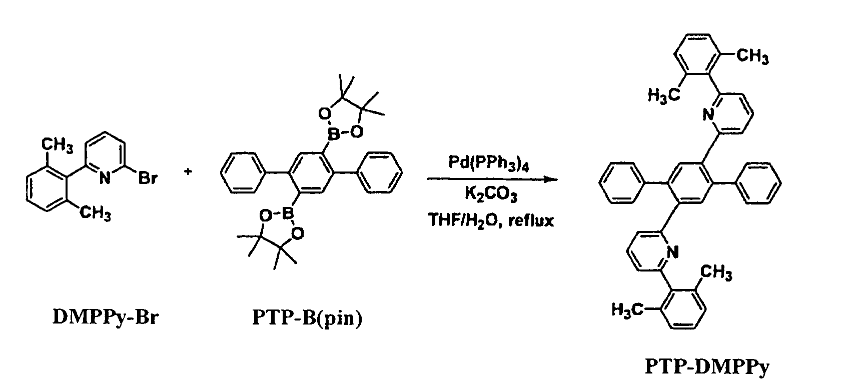

A recovery flask was charged with DMPPy-Br (1.18 g, 4.50 mmol), PTP-P(pin) (908 mg, 1.88 mmol), Pd(PPh₃)₄ (220 mg, 0.190 mmol) and K₂CO₃ (634 mg, 4.59 mmol), followed by adding thereto THF (10 mL) and H₂O (5 mL). The resulting mixture was heat-refluxed. After the completion of the reaction, the mixture was subjected to separation with dichloromethane. The separated solution was dried with Na₂SO₄, and then, subjected to cerite filtration. A crude product of PTP-DMPPy was obtained by distilling the solvent from the solution under reduced pressure. Toluene was added to the crude product, thereby extracting an insoluble component and yielding the target compound in white solid form (1.03 g, 93%). ¹H NMR (400 MHz, CD₂Cl₂): ≡ 7.72 (s, 2H), 7.54 (t, J_{HH} = 7.8 Hz, 2H), 7.30-7.23 (m, 10H), 7.19-7.15 (m, 2H), 7.08 (d, J_{HH} = 8.0 Hz, 4H), 7.65 (d, J_{HH} = 8.0 Hz, 2H), 7.01 (d, J_{HH} = 7.8 Hz, 2H), 2.00 (s, 12H). EI MS m/z 592.5 (M⁺).
As a result of property measurements, it has been verified that the produced compound had a glass transition temperature of 95°C and showed significantly superior properties to those of CBP of Comparative Example 1.

### Example 4

### Production of 1,4-bis((2',4',5',6'-tetrafluoro-3'-(3"-pyridyl))phenyl)-2,5-diphenylbenzene (PTP-Py4FP)

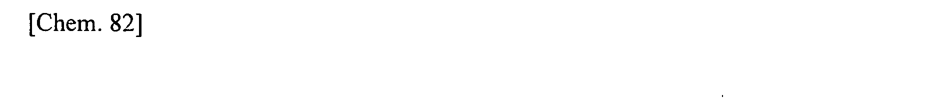

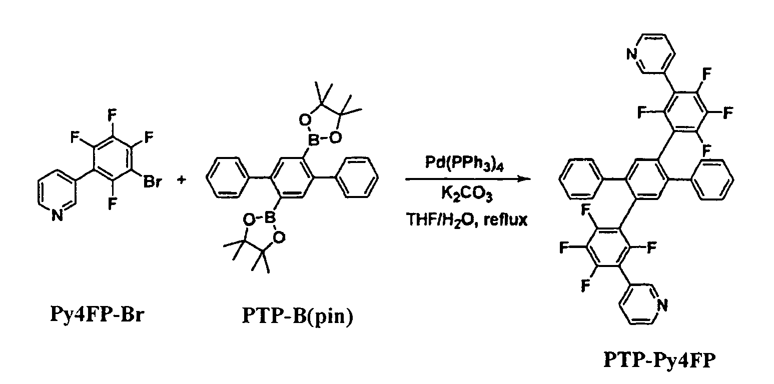

A recovery flask was charged with Py4FP-Br (1.20 g, 3.92 mmol), PTP-B(pin) (793 mg, 1.64 mmol), Pd(PPh₃)₄ (386 mg, 0.334 mmol) and K₂CO₃ (571 mg, 4.13 mmol), followed by adding thereto THF (10 mL) and H₂O (5 mL). Te resulting mixture was heat-refluxed. After the completion of the reaction, THF was distilled from the mixture. The mixture was then admixed with 7% H₂O₂ and subjected to separation with chloroform. The separated solution was dried with Na₂SO₄, and then, subjected to cerite filtration. A crude product of PTP-Py4FP was obtained by distilling the solvent from the solution under reduced pressure. The crude product was purified by preparative GPC and washed with toluene, thereby yielding the target compound in white solid form (890 g, 79%). ¹H NMR (400 MHz, CD₂Cl₂): ≡ 8.62-8.59 (m, 2H), 8.45 (s, 2H), 7.61 (s, 2H), 7.60-7.55 (m, 2H), 7.40-7.26 (m, 12H). ¹⁹F NMR (376.1 MHz, CDCl₃): ≡ -118.4 (t, J_{FF} = 9.4 Hz, 2F), -133.2 (d, J_{FF} = 22.5 Hz, 2F), -136.9 (d, J_{FF} = 22.5 Hz, 2F), -163.9 (qui, J_{FF} = 22.5 Hz, 9.4 Hz, 2H). EI MS m/z 680.4 (M⁺).
As a result of property measurements, it has been verified that the produced compound had a glass transition temperature of 80°C and a triplet excited state energy of 2.7 eV and showed significantly superior properties to those of CBP of Comparative Example 1.

### Example 5

### Production of 1,4-bis((6'-methyl-3'-(2"-methyl)phenyl)phenyl)-2,5-diphenylbenzene (PTP-MPMP)

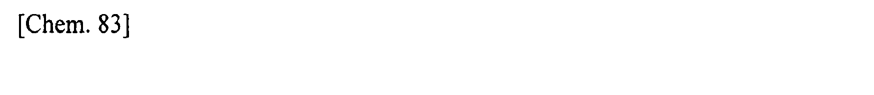

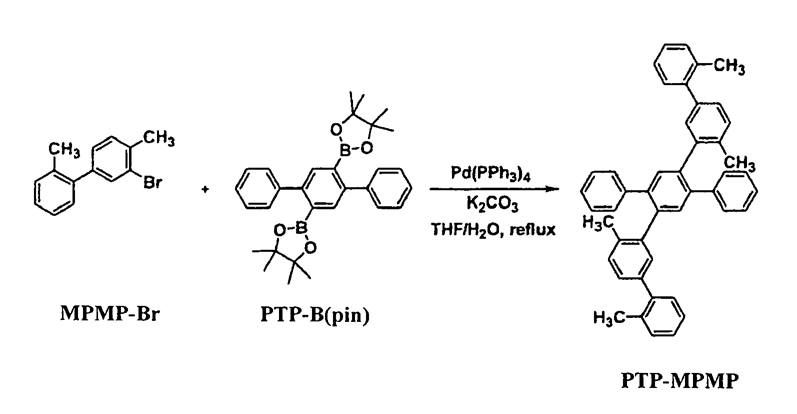

A recovery flask was charged with MPMP-Br (1.99 g, 7.65 mmol), PTP-B(pin) (1.57 g, 3.26 mmol), Pd(PPh₃)₄ (380.5 mg, 0.33 mmol) and K₂CO₃ (1.06 mg, 7.67 mmol), followed by adding thereto THF (12 mL) and H₂O (6 mL). The resulting mixture was heat-refluxed. After the completion of the reaction, THF was distilled from the mixture. The mixture was then subjected to separation with dichloromethane. The separated solution was dried with MgSO₄ and subjected to cerite filtration. A crude product of PTP-MPMP was obtained by distilling the solvent from the solution under reduced pressure. The crude product was filtered by a silica gel and purified by preparative GPC, thereby yielding the target compound in white solid form (1.36 g, 71%). ¹H NMR (400 MHz, CD₂Cl₂): ≡ 7.45 (s, 2H), 7.24-7.16 (m, 20H), 7.12 (d, J_{HH} = 8.0 Hz, 2H), 2.15 (s, 3H), 2.13 (s, 3H), 2.05 (s, 6H). EI MS m/z 590.5 (M⁺).
As a result of property measurements, it has been verified that the produced compound had a glass transition temperature of 85°C and a triplet excited state energy of 2.8 eV and showed significantly superior properties to those of CBP of Comparative Example 1.

### Example 6

### Production of 1,4-bis((6'-methyl-3'-(6"-methyl)-3"-pyridyl)phenyl)-2,5-diphenylbenzene (PTP-MPyMP)

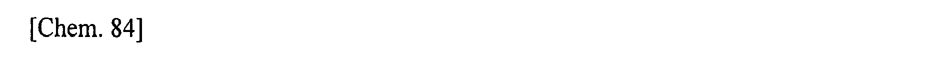

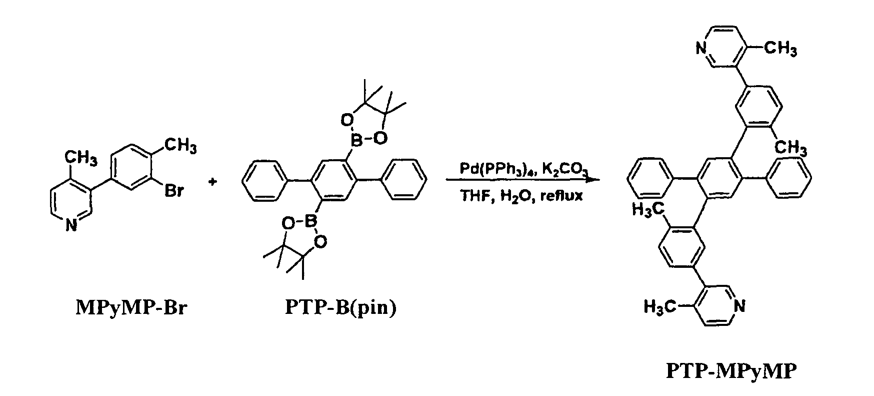

A recovery flask was charged with MPyMP-Br (1.89 g, 7.20 mg), PTP-B(pin) (1.45 g, 3.00 mmol), Pd(PPh₃)₄ (346.7 mg, 0.30 mmol) and K₂CO₃ (829.3 mg, 6.00 mmol), followed by adding thereto THF (20 mg) and H₂O (10 mg). The resulting mixture was heat-refluxed. After the completion of the reaction, the mixture was subjected to separation with toluene. The separated solution was dried with Na₂SO₄ and subjected to cerite filtration. A crude product of PTP-MPyMP was obtained by distilling the solvent from the solution under reduced pressure. Hexane was added to the crude product, thereby extracting an insoluble component and yielding the target compound in white solid form (1.69 g, 95%). ¹H NMR (400 MHz, CD₂Cl₂): ≡ 8.37 (d, J_{HH} = 5.6 Hz, 2H), 8.27 (s, 2H), 7.47 (s, 2H), 7.26-7.24 (m, 2H), 7.20 (m, 10H), 7.12-7.04 (m, 6H), 2.23 (s, 3H), 2.21 (s, 3H), 2.00 (s, 3H), 1.99 (s, 3H). EI MS m/z 592.6 (M⁺).
As a result of property measurements, it has been verified that the produced compound had a glass transition temperature of 90°C and a triplet excited state energy of 2.8 eV and showed significantly superior properties to those of CBP of Comparative Example 1.

### Example 7

### Production of 1,4-bis((6'-methyl-3'-(6"-methyl-3"-pyridyl)phenyl)-2,5-bis(2'-methylphenyl)benzene (CH3-PTP-MPyMP)

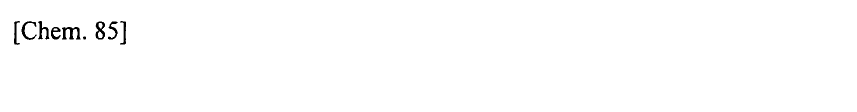

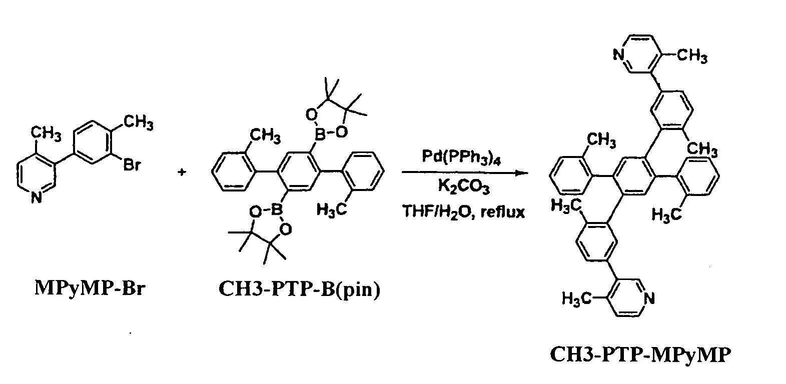

A recovery flask was charged with MPyMP-Br (1.89 g, 7.20 mmol), CH₃-PTP-B(pin) (1.45 g, 3.00 mmol), Pd(PPh₃)₄ (346 mg, 0.30 mmol) and K₂CO₃ (829 mg, 6.00 mmol), followed by adding thereto THF (20 mL) and H₂O (10 mL). The resulting mixture was heat-refluxed. After the completion of the reaction, the mixture was subjected to separation with chloroform. The separated solution was dried with Na₂SO₄ and subjected to cerite filtration. A crude product of CH₃-PTP-MPyMP was obtained by distilling the solvent from the solution under reduced pressure. Ethyl acetate was added to the crude product, thereby extracting an insoluble component and yielding the target compound in white solid form (1.45 g, 78%). ¹H NMR (400 MHz, CD₂Cl₂): ≡ 8.37 (d, J_{HH} = 4.8 Hz, 2H), 8.10 (m, 2H), 7.36 (s, 2H), 7.28-7.26 (m, 4H), 7.24-6.81 (m, 12H), 2.36-1.89 (m, 18H). EI MS m/z 620.4 (M⁺).
As a result of property measurements, it has been verified that the produced compound had a glass transition temperature of 95°C and a triplet excited state energy of 2.8 eV and showed significantly superior properties to those of CBP of Comparative Example 1.

### Example 8

### Production of 1,4-bis(2',4',6'-trimethyl-3'-(2"-pyridyl)phenyl-2,5-diphenylbenzene (PTP-2PyMS)

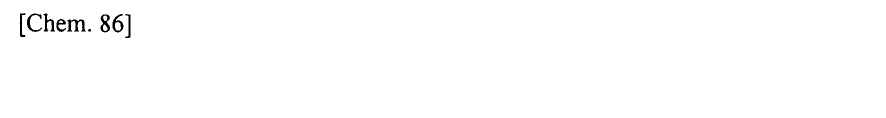

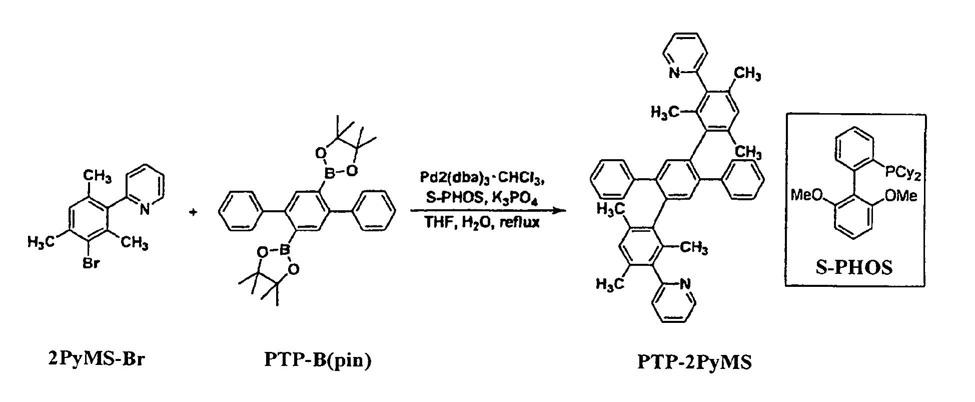

A recovery flask was charged with 2PyMS-Br (2.49 g, 9.00 mmol), PTP-B(pin) (1.45 g, 3.00 mmol), Pd₂(dba)₃·CHCl₃ (77.6 mg, 0.075 mmol), S-PHOS (123 mg, 0.30 mmol) and K₃PO₄ (1.27 g, 6.00 mmol), followed by adding thereto THF (30 mL) and H₂O (15 mL). The resulting mixture was heat-refluxed. After the completion of the reaction, the mixture was subjected to separation with toluene. The separated solution was dried with Na₂SO₄ and subjected to cerite filtration. A crude product of PTP-2PyMS was obtained by distilling the solvent from the solution under reduced pressure. The crude product was purified by sublimation, thereby yielding the target compound in white solid form (380 mg, 20%). ¹H NMR (400 MHz, CDCl₃): ≡ 8.71 (m, 2H), 7.71 (m, 2H), 7.33 (s, 2H), 7.23-7.19 (m, 12H), 7.00-6.90 (m, 12H), 1.98-1.72 (m, 18H). EI MS m/z 620.0
(M⁺).
As a result of property measurements, it has been verified that the produced compound had a glass transition temperature of 113°C and a triplet excited state energy of 2.7 eV and showed significantly superior properties to those of CBP of Comparative Example 1.

### Example 9

### Production of 1,4-bis(3',5'-bis((2"-methyl)-phenyl)phenyl)-2,5-diphenylbenzene (PTP-BMPP)

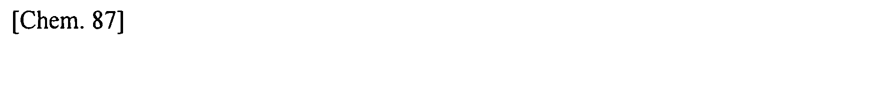

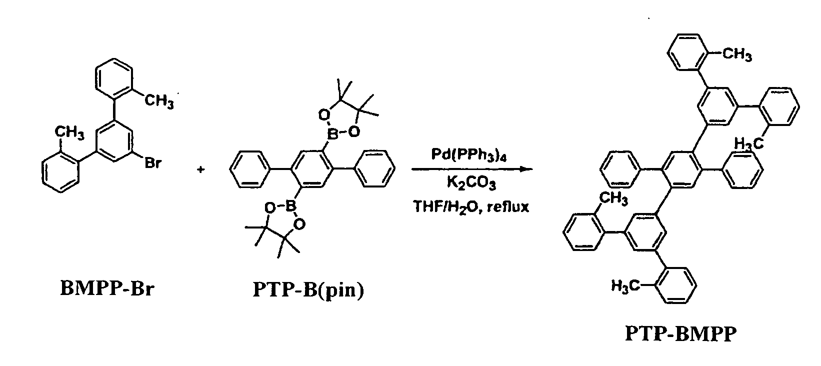

A recovery flask was charged with BMPP-Br (2.53 g, 7.50 mmol), PTP-B(pin) (1.21 g, 2.51 mmol), Pd(PPh₃)₄ (300 mg, 0.26 mmol) and K₂CO₃ (1.05 g, 7.60 mmol), followed by adding thereto THF (10 mL) and H₂O (5 mL). The resulting mixture was heat-refluxed. After the completion of the reaction, THF was distilled from the mixture. The mixture was then subjected to separation with chloroform. The separated solution was dried with MgSO₄ and subjected to cerite filtration. A crude product of PTP-BMPP was obtained by distilling the solvent from the solution under reduced pressure. Diethyl ether was added to the crude product, thereby extracting an insoluble component and yielding the target compound in white solid form (1.52 g, 82%). ¹H NMR (400 MHz, CD₂Cl₂): ≡ 7.61 (s, 2H), 7.34-7.26 (m, 10H), 7.21-7.11 (m, 18H), 7.10 (d, J_{HH} = 7.2 Hz, 4H), 2.14 (s, 12H). EI MS m/z 742.7 (M⁺).
As a result of property measurements, it has been verified that the produced compound had a glass transition temperature of 110°C and a triplet excited state energy of 2.8 eV and showed significantly superior properties to those of CBP of Comparative Example 1.

### Example 10

### Production of 1,4-bis((3',5'-bis((2"-methyl)-phenyl)phenyl)-2,5-bis(2'-methylphenyl)benzene (CH3-PTP-BMPP)

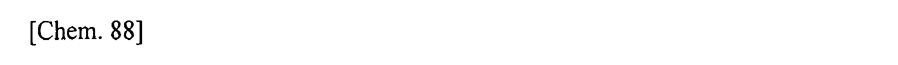

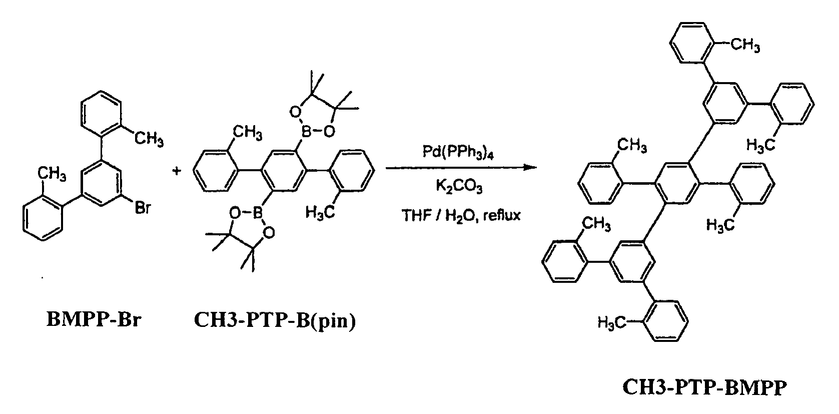

A recovery flask was charged with BMPP-Br (3.06 g, 9.07 mmol), CH3-PTP-B(pin) (1.56 g, 3.06 mmol), Pd(PPh₃)₄ (354 mg, 0.306 mmol) and K₂CO₃ (1.26 g, 9.12 mmol), followed by adding thereto THF (12 mL) and H₂O (6 mL). The resulting mixture was heat-refluxed. After the completion of the reaction, THF was distilled from the mixture. The mixture was then subjected to separation with dichloromethane. The separated solution was dried with MgSO₄ and subjected to cerite filtration. A crude product of CH3-PTP-BMPP was obtained by distilling the solvent from the solution under reduced pressure. Diethyl ether was added to the crude product, thereby extracting an insoluble component and yielding the target compound in white solid form (1.82 g, 77%). ¹H NMR (400 MHz, CD₂Cl₂): ≡ 7.48 (d, J_{HH} = 4.0 Hz, 2H), 7.32 (d, J_{HH} = 7.2 Hz, 2H), 7.23-7.14 (m, 22H), 7.08 (s, 2H), 6.99 (d, J_{HH} = 7.2 Hz, 4H), 2.11 (s, 12H), 2.04 (s, 6H). EI MS m/z 770.8 (M⁺).
As a result of property measurements, it has been verified that the produced compound had a glass transition temperature of 110°C and a triplet excited state energy of 2.9 eV and showed significantly superior properties to those of CBP of Comparative Example 1.

### Example 11

### Production of 1,4-bis(3',5'-bis((6"-methyl)-3"-pyridyl)phenyl)-2,5-diphenylbenzene (PTP-BMPyP)

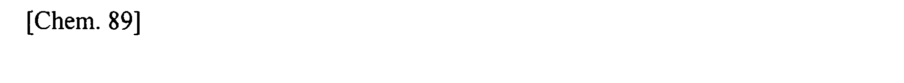

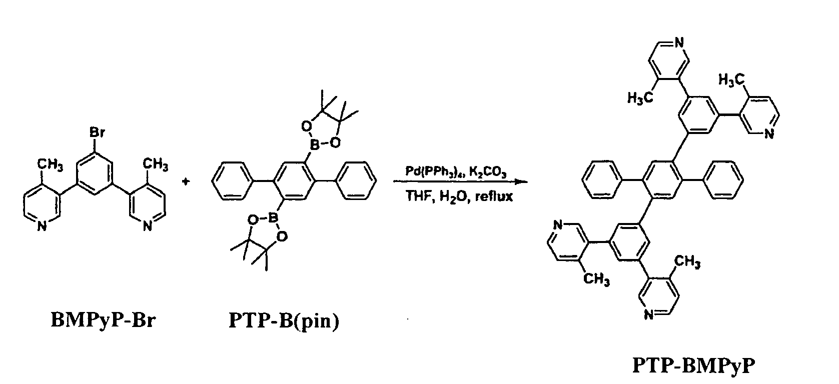

A recovery flask was charged with BMPyP-Br (2.04 g, 6.00 mmol), PTP-B(pin) (1.21 g, 2.50 mmol), Pd(PPh₃)₄ (289 mg, 0.25 mmol) and K₂CO₃ (691 mg, 5.00 mmol), followed by adding thereto THF (20 mL) and H₂O (10 mL). The resulting mixture was heat-refluxed. After the completion of the reaction, the mixture was subjected to separation with chloroform. The separated solution was dried with Na₂SO₄, and then subjected to cerite filtration. A crude product of PTP-BMPyP was obtained by distilling the solvent from the solution under reduced pressure. Acetonitrile was added to the crude product, thereby extracting an insoluble component and yielding the target compound in white solid form (1.63 g, 87%). ¹H NMR (400 MHz, CD₂Cl₂): ≡ 8.43 (d, J_{HH} = 5.6 Hz, 4H), 8.28 (s, 4H), 7.61 (s, 2H), 7.31 (m, 10H), 7.24 (d, J_{HH} = 8.0 Hz, 2H), 7.15-7.14 (m, 6H), 2.15 (s, 12H). EI MS m/z 746.0 (M⁺).
As a result of property measurements, it has been verified that the produced compound had a glass transition temperature of 125°C and a triplet excited state energy of 2.8 eV and showed significantly superior properties to those of CBP of Comparative Example 1.

### Example 12

### Production of 1,4-bis(3',5'-bis((6"-methyl)-3"-pyridyl)phenyl)-2,5-bis(2'-methylphenyl)benzene (CH3-PTP-BMPyP)

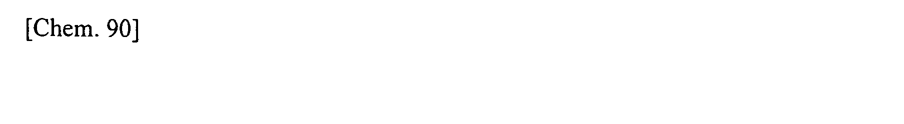

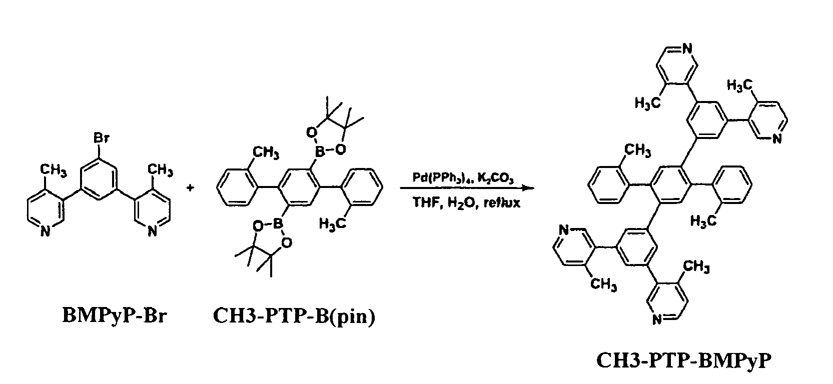

A recovery flask was charged with BMPyP-Br (2.04 g, 6.00 mmol), CH3-PTP-B(pin) (1.28 g, 2.50 mmol), Pd(PPh₃)₄ (289 mg, 0.25 mmol) and K₂CO₃ (691 mg, 5.00 mmol), followed by adding thereto THF (20 mL) and H₂O (10 mL). The resulting mixture was heat-refluxed. After the completion of the reaction, the mixture was subjected to separation with chloroform. The separated solution was dried with Na₂SO₄ and subjected to cerite filtration. A crude product of PTP-BMPyP was obtained by distilling the solvent from the solution under reduced pressure. Acetonitrile was added to the crude product, thereby extracting an insoluble component and yielding the target compound in white solid form (1.87 g, 95%). ¹H NMR (400 MHz, CD₂Cl₂): ≡ 7.72 (s, 2H), 7.54 (t, J_{HH} = 7.8 Hz, 2H), 7.30-7.23 (m, 10H), 7.19-7.15 (m, 2H), 7.08 (d, J_{HH} = 8.0 Hz, 4H), 7.65 (d, J_{HH} = 8.0 Hz, 2H), 7.01 (d, J_{HH} = 7.8 Hz, 2H), 2.00 (s, 12H). EI MS m/z 775.0 (M⁺).
As a result of property measurements, it has been verified that the produced compound had a glass transition temperature of 120°C and a triplet excited state energy of 2.8 eV and showed significantly superior properties to those of CBP of Comparative Example 1.

### [Comparative Example 1]

A known material, CBP, had no glass transition temperature, poor film stability and the characteristic of being easy to crystallize. Further, the triplet excited state of CBP was 2.6 eV.

### [Evaluations of Blue Phosphorescent Organic Electroluminescent Devices]

### Example 13

A transparent electrode of ITO was formed with a thickness of 110 nm on a glass substrate, followed by ultrasonic cleaning with a cleaner, ultrasonic cleaning with pure water, ultrasonic cleaning twice with acetone, washing with isopropyl alcohol, boil washing with isopropyl alcohol, and then, drying. The electrode was subsequently subjected to UV ozone treatment. The resulting substrate assembly was immediately introduced into a vacuum chamber.

By vacuum deposition (vacuum degree: 10⁻⁴ Pa), 40 nm of α-NPD was deposited as a positive hole transfer layer. Next, 10 nm of mCP was deposited as an exciton block layer. A light-emitting layer was then formed with a thickness of 20 nm by simultaneously vapor depositing the PTP-PyMS produced in Example 1 as a host material and iridium complex (FIrpic) as a dopant material in such a manner that the percentage of the dopant material relative to the host material was 6% by weight. Further, 40 nm of BPhen was deposited as a positive hole block layer and as an electron transfer layer. After that, a metal electrode was formed with a thickness of 100 nm by simultaneously depositing Mg and Ag at a weight ratio of 10:1. Thereafter, 10 nm of Ag was deposited as a protection layer. With this, an organic electroluminescent device was obtained.

The light emission efficiency and emission spectrum of the thus-obtained organic electroluminescent device were measured by driving the device continuously with the application of a direct current. The emission of blue light (peak wavelength: 470 nm) from the blue phosphorescent material (FIrpic) was observed. Further, the external quantum efficiency of the device was 10% and was significantly higher than that of Comparative Example 2 using CBP as a host material under the completely same conditions (as explained later).

### Example 14

An organic electroluminescent device was produced in the same manner as in Example 13, except for using the PTP-PMS as the host material. The emission of blue light (peak wavelength: 470 nm) from the blue phosphorescent material (FIrpic) was observed. The external quantum efficiency of the device was 7.3% and was significantly higher than that of Comparative Example 2 using CBP as the host material under the completely same conditions (as explained later).

### Example 15

An organic electroluminescent device was produced in the same manner as in Example 13, except for using the PTP-MPyMP as the host material. The emission of blue light (peak wavelength: 470 nm) from the blue phosphorescent material (FIrpic) was observed. The external quantum efficiency of the device was 8.4% and was significantly higher than that of Comparative Example 2 using CBP as the host material under the completely same conditions (as explained later).

### Example 16

An organic electroluminescent device was produced in the same manner as in Example 13, except for using the CH3-PTP-MPyMP as the host material. The emission of blue light (peak wavelength: 470 nm) from the blue phosphorescent material (FIrpic) was observed. The external quantum efficiency of the device was 7.0% and was significantly higher than that of Comparative Example 2 using CBP as the host material under the completely same conditions (as explained later).

### Example 17

An organic electroluminescent device was produced in the same manner as in Example 13, except for using the PTP-2PyMS as the host material. The emission of blue light (peak wavelength: 470 nm) from the blue phosphorescent material (FIrpic) was observed. The external quantum efficiency of the device was 6.9% and was significantly higher than that of Comparative Example 2 using CBP as the host material under the completely same conditions (as explained later).

### Example 18

An organic electroluminescent device was produced in the same manner as in Example 13, except for using the PTP-BMPP as the host material. The emission of blue light (peak wavelength: 470 nm) from the blue phosphorescent material (FIrpic) was observed. The external quantum efficiency of the device was 7.3% and was significantly higher than that of Comparative Example 2 using CBP as the host material under the completely same conditions (as explained later).

### Example 19

An organic electroluminescent device was produced in the same manner as in Example 13, except for using the CH3-PTP-BMPP as the host material. The emission of blue light (peak wavelength: 470 nm) from the blue phosphorescent material (FIrpic) was observed. The external quantum efficiency of the device was 9.6% and was significantly higher than that of Comparative Example 2 using CBP as the host material under the completely same conditions (as explained later).

### Example 20

An organic electroluminescent device was produced in the same manner as in Example 13, except for using the PTP-BMPyP as the host material. The emission of blue light (peak wavelength: 470 nm) from the blue phosphorescent material (FIrpic) was verified. The external quantum efficiency of the device was 10% and was significantly higher than that of Comparative Example 2 using CBP as the host material under the completely same conditions (as explained later).

### Example 21

An organic electroluminescent device was produced in the same manner as in Example 13, except for using the CH3-PTP-BMPyP as the host material. The emission of blue light (peak wavelength: 470 nm) from the blue phosphorescent material (FIrpic) was observed. The external quantum efficiency of the device was 9.1 % and was significantly higher than that of Comparative Example 2 using CBP as the host material under the completely same conditions (as explained later).

### [Comparative Example 2]

An organic electroluminescent device was produced in the same manner as in Example 13, except for using CBP as the host material. The external quantum efficiency of the device was 5.7% and was inferior to those of Examples 13 to 21.

### Example 22

The organic electroluminescent device of Example 14 was driven with a load of 100 mA/cm². At this time, the external quantum efficiency of the device was 3.8% and was significantly higher than that of Comparative Example 3 using CBP as the host material. It has thus been shown that the host material high durability.

### Example 23

The organic electroluminescent device of Example 18 was driven with a load of 100 mA/cm². At this time, the external quantum efficiency of the device was 3.8% and was significantly higher than that of Comparative Example 3 using CBP as the host material. It has been shown that the host material had high durability.

### Example 24

The organic electroluminescent device of Example 19 was driven with a load of 100 mA/cm². At this time, the external quantum efficiency of the device was 4.9% and was significantly higher than that of Comparative Example 3 using CBP as the host material. It has been shown that the host material had high durability.

### [Comparative Example 3]

The organic electroluminescent device of Comparative Example 2 was driven with a load of 100 mA/cm². The external quantum efficiency of the device was 3.0% and was inferior to those of Examples 22 to 24.

As described above, it has been confirmed that the oligophenylene material of the present invention effectively serves as a host material for blue phosphorescent emission and contributes to a significantly higher EL device efficiency than conventional blue phosphorescent host material CBP (carbazole host material) and previously invented benzoazole compounds. This means that the host material of the present invention can attain a sufficient band gap for blue phosphorescent emission and cause energy transition efficiently in the light-emitting device by the synergistic effect of: (1) increasing the flexibility (amorphous nature) of the molecule due to the twisted molecular structure and thereby improving the durability of the light-emitting layer; and (2) weakening the π conjugation of the molecule due to the non-planarity of the molecular structure and thereby increasing the band gap of the material effectively.

## Claims

1. An oligophenylene derivative represented by the general formula (1) 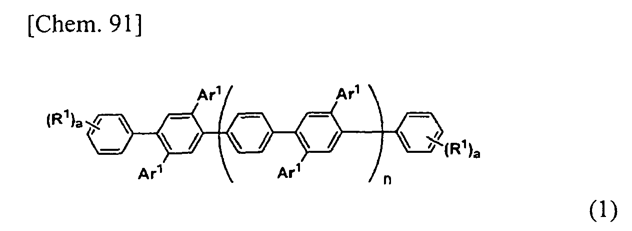 where Ar¹ each independently represents an oligophenyl group of the following formula (1a); *n* is 0 or 1; R¹ each independently represents an C₁-C₆ alkyl group, a C₁-C₆ fluoroalkyl group or a halogen atom; and *a* is each independently an integer of 0 to 5
[Chem. 92] -(Ar')_{b}-(Ar")_{c} (1a)
where Ar' each independently represents a divalent to hexavalent aromatic ring which may have a substituent or substituents; Ar" each independently represents a phenyl group which may have a substituent or substituents; each substituent of Ar' and Ar" can be located at any position on the aromatic ring and is selected from the group consisting of a C₁-C₆ alkyl group, a C₁-C₆ fluoroalkyl group and a halogen atom; each of Ar' and Ar" may have one to four nitrogen atoms as a heteroatom in the aromatic ring; *b* is an integer of 1 to 4; c is an integer of 1 to 5; Ar' and Ar' or Ar' and Ar" are bonded to each other by a single C-C bond between carbon atoms of the respective aromatic rings; c number of Ar" can be bonded to Ar' in straight chain form or in branched chain form or can be directly bonded to Ar'.

2. The oligophenylene derivative according to claim 1, wherein Ar¹ is an oligophenyl group of the following formula (Ib)
[Chem. 93] - (Ar') _{b}- (Ar") (1b)
where Ar', Ar" and *b* are the same as defined in the formula (1a).

3. The oligophenylene derivative according to claim 1, wherein Ar¹ is an oligophenyl group of the following formula (1c) 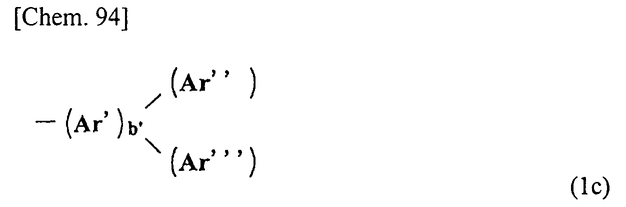 where Ar' and Ar" are the same as defined in the formula (1a); A"' is the same as Ar"; and *b'* is the same as *b.*

4. The oligophenylene derivative according to any one of claims 1 to 3, wherein the oligophenylene derivative is a terphenyl derivative of the general formula (2) 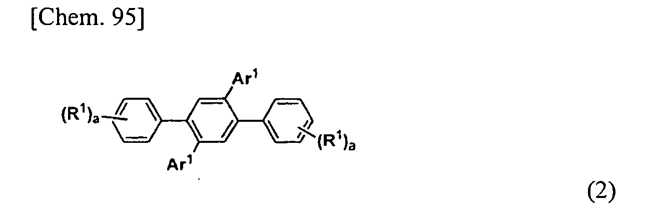 where Ar¹ and R¹ are the same as defined in the formula (1).

5. The terphenyl derivative according to claim 4, wherein the terphenyl derivative is represented by the general formula (3) 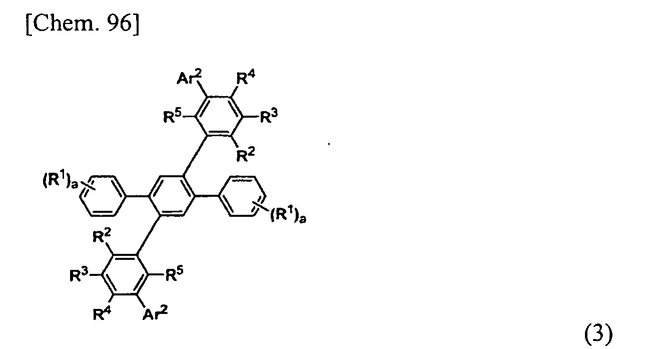 where R¹ and *a* are the same as defined in the formula (1); R², R³, R⁴ and R⁵ each independently represent a C₁-C₆ alkyl group, a C₁-C₆ fluoroalkyl group or a halogen atom; Ar² represents a phenyl group which may have a substituent or substituent; each substituent of Ar² can be located at any position on the aromatic ring and is selected from the group consisting of a C₁-C₆ alkyl group, a C₁-C₆ fluoroalkyl group and a halogen atom; and Ar² may have one to four nitrogen atoms as a heteroatom in the aromatic ring.

6. The terphenyl derivative according to claim 4, wherein the terphenyl derivative is represented by the general formula (4) 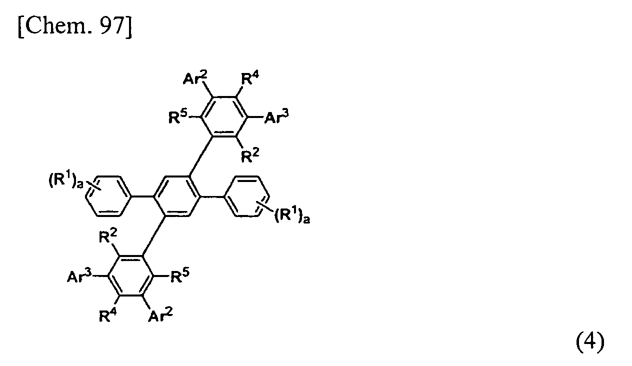 where R¹ and *a* are the same as defined in the formula (1); R², R⁴ and R⁵ each independently represent a C₁-C₆ alkyl group, a C₁-C₆ fluoroalkyl group or a halogen atom; Ar² and Ar³ each independently represent a phenyl group which may have a substituent or substituents; each substituent of Ar² and Ar³ can be located at any position on the aromatic ring and is selected from the group consisting of a C₁-C₆ alkyl group, a C₁-C₆ fluoroalkyl group and a halogen atom; and each of Ar² and Ar³ may have one to four nitrogen atoms as a heteroatom in the aromatic ring.

7. The terphenyl derivative according to claim 4, wherein the terphenyl derivative is represented by the general formula (5) 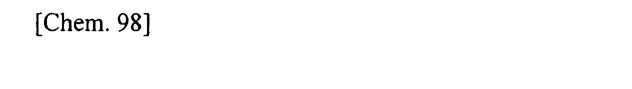 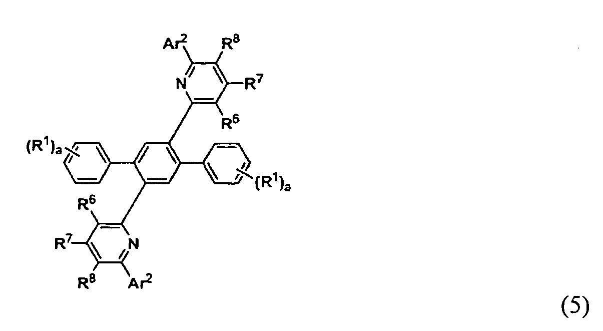 where R¹ and *a* are the same as defined in the formula (1); Ar² is the same as defined in the formula (3); and R⁶, R⁷ and R⁸ each independently represent a C₁-C₆ alkyl group, a C₁-C₆ fluoroalkyl group or a halogen atom.

8. The terphenyl derivative according to claim 4, wherein the terphenyl derivative is represented by the general formula (6) 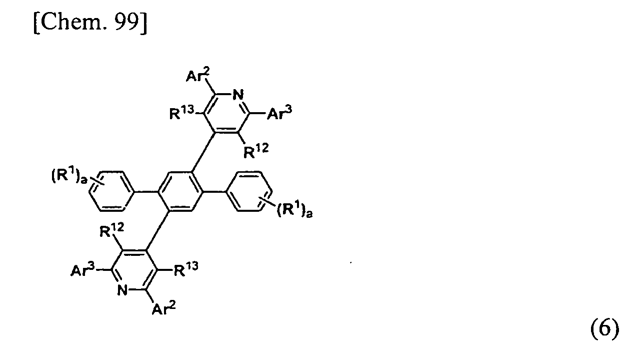 where R¹ and *a* are the same as defined in the formula (1); Ar² and Ar³ are the same as defined in the formula (5); and R¹² and R¹³ each independently represent a C₁-C₆ alkyl group, a C₁-C₆ fluoroalkyl group or a halogen atom.

9. An organic electroluminescent device, comprising a pair of electrodes and at least one organic light-emitting layer arranged between the electrodes, wherein the organic light-emitting layer contains an oligophenylene derivative represented by the following general formula (1) 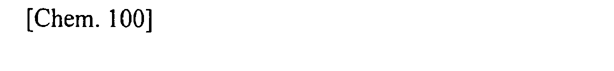 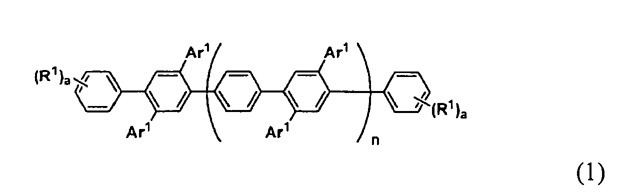 where Ar¹, R¹, *a* and *n* are the same as defined in the above formula (1).

10. The organic electroluminescent device according to claim 9, wherein Ar¹ is an oligophenyl group of the formula (1b)
[Chem. 10] -(Ar')_{b}-(Ar") (1b)
where Ar', Ar" and *b* are the same as defined in the above formula (1a).

11. The organic electroluminescent device according to claim 9, wherein Ar¹ is an oligophenyl group of the formula (1c) 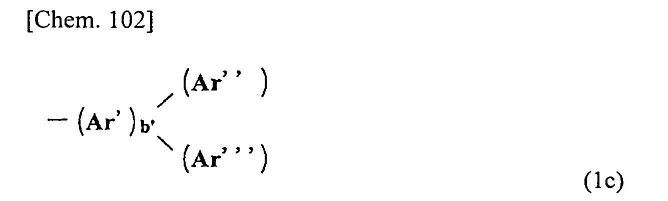 where Ar' and Ar" are the same as defined in the above formula (1a); A"' is the same as Ar"; and *b*' is the same as *b*.

12. The organic electroluminescent device according to any one of claims 9 to 11, wherein the organic light-emitting layer contains a host material and a blue phosphorescent dopant material; and the host material is the oligophenylene derivative.

13. The organic electroluminescent device according to claim 12, further comprising a positive hole transfer layer, an exciton block layer and an electron transfer layer.

14. The organic electroluminescent device according to any one of claims 9 to 13, wherein the oligophenylene derivative is a terphenyl derivative represented by the following general formula (2) 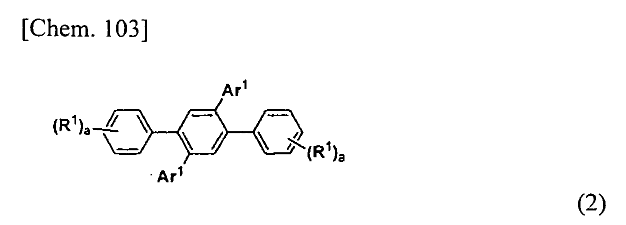 where Ar¹, R¹ and *a* are the same as defined in the above formula (2).

15. The organic electroluminescent device according to claim 14, wherein the terphenyl derivative is represented by the following general formula (3) 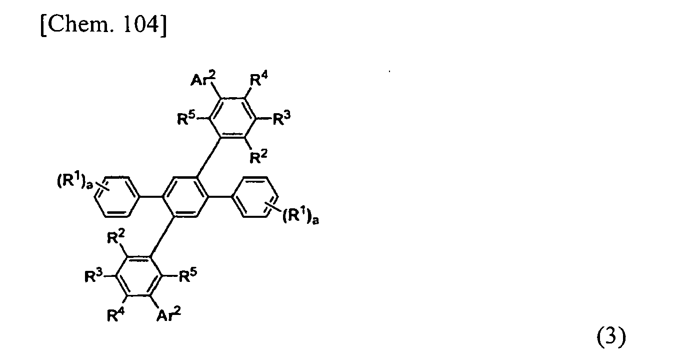 where R¹, R², R³, R⁴, R⁵, Ar² and *a* are the same as defined in the above formula (3).

16. The organic electroluminescent device according to claim 14, wherein the terphenyl derivative is represented by the following general formula (4) 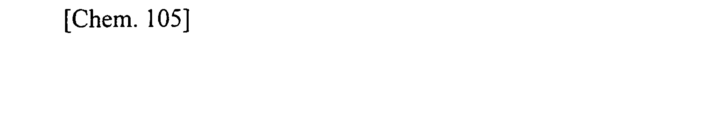 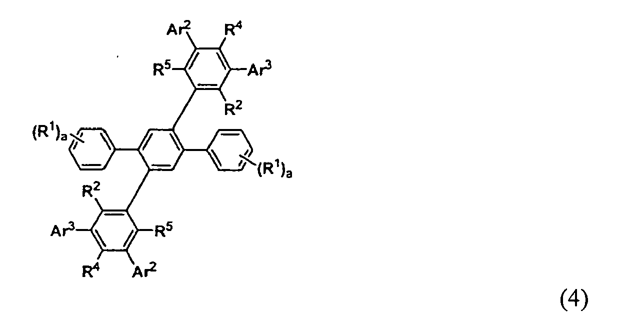 where R¹, R², R³, R⁴, R⁵, *a*, Ar² and Ar³ are the same as defined in the above formula (4).

17. The organic electroluminescent device according to claim 14, wherein the terphenyl derivative is represented by the following general formula (5) 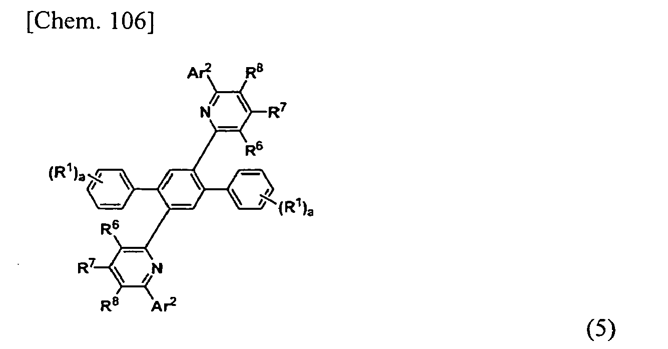 where R¹, *a*, Ar², R⁶, R⁷ and R⁸ are the same as defined in the above formula (4).

18. The organic electroluminescent device according to claim 14, wherein the terphenyl derivative is represented by the following general formula (6) 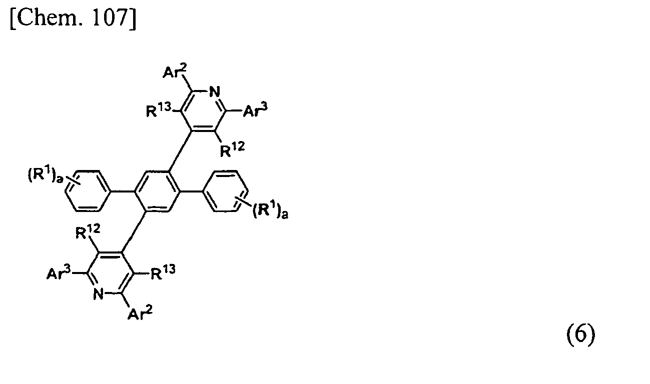 where R¹, R¹², R¹³, Ar², Ar³ and *a* are the same as defined in the above formula (6).

19. The organic electroluminescent device according to claim 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (3) 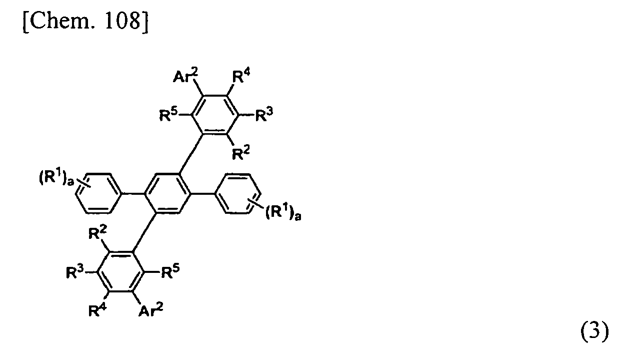 where R¹, *a*, R², R³, R⁴, R⁵ and Ar³ are the same as defined in the above formula (3); and wherein the blue phosphorescent dopant material is FIrpic represented by the following formula

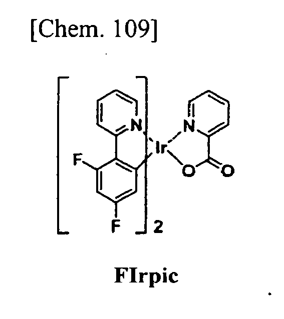

20. The organic electroluminescent device according to claim 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (4) 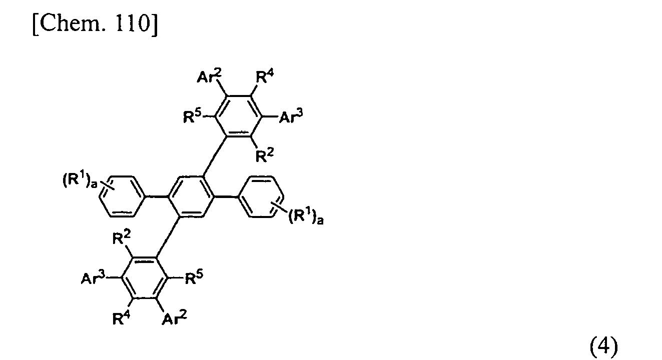 where R¹, R², R³, R⁴, R⁵, *a*, Ar² and Ar³ are the same as defined in the above formula (4); and wherein the blue phosphorescent dopant material is FIrpic represented by the following formula

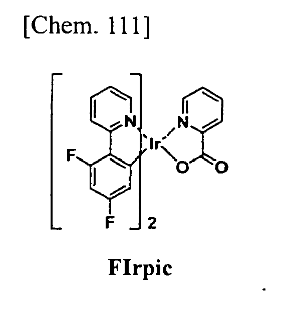

21. The organic electroluminescent device according to Feature 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (5) 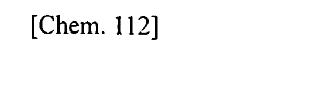 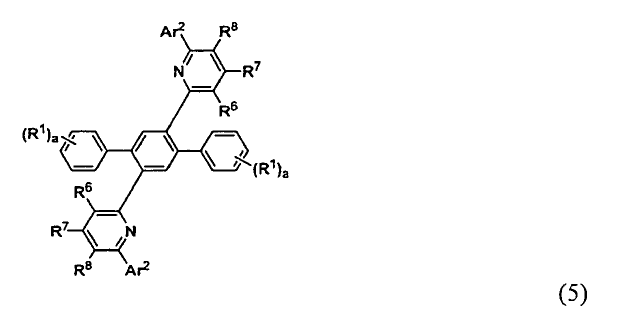 where R¹, *a*, Ar², R⁶, R⁷ and R⁸ are the same as defined in the above formula (5); and wherein the blue phosphorescent dopant material is FIrpic represented by the following formula

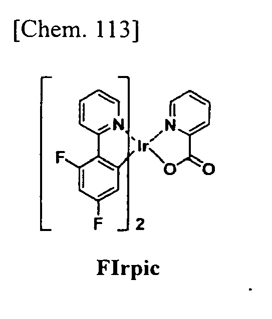

22. The organic electroluminescent device according to claim 14, wherein the organic electroluminescent device is a blue phosphorescent organic electroluminescent device having a pair of electrodes, at least one organic light-emitting layer arranged between the electrodes, a positive hole transfer layer, an exciton block layer, an electron transfer layer and a positive hole block layer; the organic light-emitting layer contains a host material and a blue phosphorescent dopant material; the host material is a compound represented by the following formula (6) 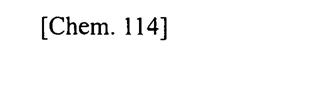 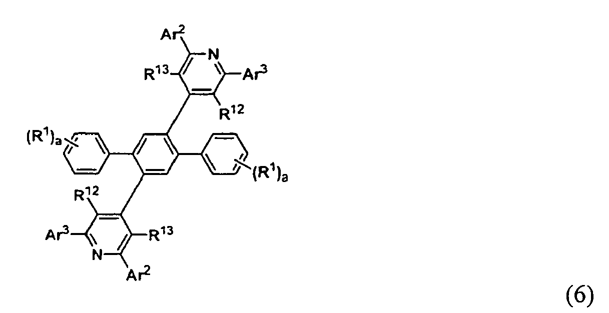 where R¹, R¹², R¹³, Ar², Ar³ and *a* are the same as defined in the above formula (6); and wherein the blue phosphorescent dopant material is FIrpic represented by the following formula
